# EUROPEAN PATENT APPLICATION

(11) **EP 2 261 242 A1**
(43) Date of publication of application: **15.12.2010**
(21) Application number: 09162450.2
(22) Date of filing: 10.06.2009
(51) Int. Cl.: C07K 14/435, C12N 9/10

(54) **Aspartate-N-acetyltransferase enzyme, diagnostic method and therapeutic method**

(71) Applicant: UNIVERSITE CATHOLIQUE DE LOUVAIN, 1348 Louvain la Neuve (BE)
(72) Inventor: Van Schaftingen, Emile, 1160 Auderghem (BE); Wiame, Elsa, 1410 Waterloo (BE)
(74) Representative: Cabinet Plasseraud

(57) **Abstract**

The present invention relates to an isolated human aspartate-N-acetyltransferase having for sequence SEQ ID NO: 1, a method for diagnosing an aspartate-N-acetyltransferase -linked disease, a kit for carrying out said method and method for treating aspartate-N-acetyltransferase-disease.

## Description

### FIELD OF THE INVENTION

The present invention relates to the molecular identification of the aspartate-N-acetyltransferase (Asp-NAT) enzyme and its use in diagnostic and drug screening.

### BACKGROUND OF THE INVENTION

N-acetylaspartate, or NAA, is the acetylated form of the amino acid aspartate, and it is present exclusively in the nervous system. Indeed, NAA is one of the most highly concentrated chemicals found in the brain of humans and animals, and yet the functions served by this brain-specific metabolite remain elusive, and controversial.

NAA is not degraded in neurons, but it is transferred by an undefined mechanism to oligodendrocytes, the myelin-forming cells, where it is hydrolyzed by aspartoacylase (aminoacylase II). The released acetate serves as a precursor for the synthesis of fatty acids, required for the formation of myelin lipids (9). NAA serves also as a precursor for N-acetylaspartylglutamate (10,11), the most abundant dipeptide present in brain and possibly a neurotransmitter (12). Other proposed roles for NAA are osmoregulation or disposal of amino groups resulting from glutamate transamination, thereby preventing the formation of ammonia from glutamate (1). Except that NAA does not appear to be a neurotransmitter (13), no agreement exists on the relative importance of these roles.

The acetyl protons on NAA give off a very prominent signal in water-suppressed, proton magnetic resonance spectroscopy (MRS), which permits clinicians to monitor levels of NAA in the brains of patients in a non-invasive manner. Because NAA is found primarily in neurons, and because the levels in the brain have been found to change rapidly after injury, or slowly during neurodegenerative diseases, MRS has become a preferred method of analyzing nerve cell dysfunction and death without surgical intervention. NAA is in particular used to monitor neuronal density and health in neurological disorders such as Alzheimer's disease, multiple sclerosis and AIDS encephalopathy (1).

NAA had also attracted attention in recent years as NAA metabolism is disrupted in two inborn errors of metabolism. Deficiency of aspartoacylase, caused by mutations in the *ASPA* gene, gives rise to a leukodystrophy known as Canavan disease (MIM 271900), which is characterized by onset in early infancy, dystonia, severe mental defect, blindness, megalencephaly, and death by 18 months on the average (14). The genetic mutation results in a defect in the enzyme that normally de-acetylates NAA in brain and in kidneys, resulting in a significant rise in NAA levels in the brain and urine.

The "opposite" disorder, i.e. the absence of brain NAA, has been described in only one patient, with truncal ataxia, marked developmental delay, seizures and secondary microcephaly (15-17). This disorder, designated "hypoacetylaspartia", is presumably due to a deficiency of Asp-NAT, but this has not been demonstrated.

NAA is formed from aspartate and acetyl-CoA by aspartate N-acetyltransferase (Asp-NAT), a membrane-bound enzyme (Goldstein, 1959, Knizley, 1967; Goldstein, 1969), which is unstable in the presence of detergents (Madhavarao and Namboodiri, 2006) and could therefore not be purified. As the molecular identification of enzymes most often involves their purification to (near-)homogeneity and the sequencing of peptides derived from the purified protein, the fact that aspartate N-acetyltransferase cannot be purified prevented its molecular identification. This molecular identification of aspartate N-acetyltransferase is important to understand the function of this enzyme, the function of NAA and the implication of this enzymatic reaction in the pathophysiology of neurological disorders.

There is therefore a need for molecularly identifying the aspartate-N-acetyltransferase (Asp-NAT) enzyme.

### SUMMARY OF THE INVENTION

One object of the invention is an isolated human aspartate-N-acetyltransferase having for sequence SEQ ID NO: 1 or an amino acid sequence having at least 90% identity with SEQ ID NO: 1.

Another object of the invention is an isolated murine aspartate-N-acetyltransferase having for sequence SEQ ID NO: 2 or an amino acid sequence having at least 90% identity with SEQ ID NO: 2.

Another object of the invention is an isolated rat aspartate-N-acetyltransferase having for sequence SEQ ID NO: 3 or an amino acid sequence having at least 90% identity with SEQ ID NO: 3.

Another object of the invention is an isolated nucleic acid sequence encoding the aspartate-N-acetyltransferase as described here above and fragments thereof.

In one embodiment of the invention, said nucleic acid sequence encodes the human aspartate-N-acetyltransferase of claim 1 and has for sequence SEQ ID NO: 4.

Another object of the invention is a vector comprising a nucleic acid sequence as described here above.

Another object of the invention is a host cell comprising a nucleic acid sequence as described here above or a vector as described here above.

Another object of the invention is a non-human transgenic animal for aspartate-N-acetyltransferase.

Another object of the invention is an antibody that specifically recognises aspartate-N-acetyltransferase as described here above.

Another object of the invention is a method for screening or identifying compounds capable of modulating the aspartate-N-acetyltransferase enzyme activity, comprising
- contacting a compound to be tested with a model system expressing the aspartate-N-acetyltransferase enzyme having for sequence SEQ ID NO: 1, SEQ ID NO: 2 or SEQ ID NO: 3 in conditions allowing the enzymatic reaction, and
- determining the aspartate-N-acetyltransferase enzyme activity.

Another object of the invention is a method for determining whether a subject has or is at risk of having an aspartate-N-acetyltransferase enzyme-linked disease in a subject in need thereof, comprising detecting in a sample obtained from said subject genetic lesions or mutations in a nucleic acid sequence as described here above.

In one embodiment of the invention, said aspartate-N-acetyltransferase enzyme activity-linked disease is selected in the group consisting of hypoacetylaspartia (aspartate N-acetyltransferase deficiency), epilepsy, canavan disease, leukodystrophies, Alzheimer's disease, multiple sclerosis, amyotrophic lateral sclerosis, stroke, AIDS encephalopathy and herpes encephalitis.

Another object of the invention is a kit comprising at least three couples of primers that allow the amplification of each of the three exons of the aspartate-N-acetyltransferase. Another object of the invention is aspartate for use in treating aspartate-N-acetyltransferase linked disease wherein the level of NAA is decreased.

Another object of the invention is N-acetylaspartate (NAA) or precursor thereof for use in treating aspartate-N-acetyltransferase linked disease wherein no NAA is produced.

### DETAILED DESCRIPTION OF THE INVENTION

As a result of their research, the inventors found the molecular identity of the aspartate-N-acetyltransferase that catalyses NAA synthesis. In addition, the inventors showed that said enzyme is mutated in primary NAA deficiency (hypoacetylaspartia), and thus provide a method for diagnosing said disease.

One object of the invention is the isolated human aspartate-N-acetyltransferase having for sequence SEQ ID NO: 1 or an amino acid sequence having at least 90% identity with SEQ ID NO: 1, preferably at least 91%, 92%, 93%, 94%, 95%, 96%, 97%, 98% and more preferably at least 99% identity with SEQ ID NO: 1.

SEQ ID NO: 1 is disclosed under EAW82541 NCBI accession number. EAW82541 discloses a sequence encoding a hypothetical protein LOC339983. The only information known about this hypothetical protein is that it comprises a GCN5-related N-acetyltransferases (GNAT) region (amino acids 184-265), which is representative of a large superfamily of functionally diverse enzymes that catalyze the transfer of an acetyl group from acetyl-Coenzyme A to the primary amine of a wide range of acceptor substrates.

Said large superfamily of N-acetyltransferases comprises about 30 genes identified in the human genome, among which about 15 are well identified.

The function of the putative protein disclosed in EAW82541 was thus unknown.

As shown in the Examples, the inventors provide the evidence that the protein having for sequence SEQ ID NO: 1 is the aspartate-N-acetyltransferase that catalyses NAA synthesis in brain.

According to the invention, to determine the percent identity of two amino acids sequences or nucleic acids sequences, the sequences are aligned for optimal comparison purposes (e.g. gaps can be introduced in the sequence of the first sequence for optimal alignment with the second sequence). The percent identity between the two sequences is function of the number of identical positions shared by the sequences. In one embodiment, the sequences to be compared are about of the same length. In another embodiment, the lengths of the sequences to be compared are the same -/+ 10%. The determination of percent identity between two sequences can be accomplished using a mathematical algorithm. Examples of algorithms that can be used include, but are not limited to, BLAST, FASTA...

According to the invention, the amino acid sequence that has at least 90% identity with SEQ ID NO: 1 conserves a function identical to SEQ ID NO: 1 function. Said function can be assessed by expressing the sequence to be tested in HEK293T cells and quantification of NAA synthesis as described in the Examples.

Another object of the invention is an isolated murine aspartate-N-acetyltransferase having for sequence SEQ ID NO: 2 or an amino acid sequence having at least 90% identity with SEQ ID NO: 2, preferably at least 91%, 92%, 93%, 94%, 95%, 96%, 97%, 98% and more preferably at least 99% identity with SEQ ID NO: 2.

SEQ ID NO: 2 is disclosed under NM_001001985.3 NCBI accession number. The only information known about this hypothetical protein is that it comprises a GCN5-related N-acetyltransferases (GNAT) region (amino acids 184-265), which is representative of a large superfamily of functionally diverse enzymes that catalyze the transfer of an acetyl group from acetyl-Coenzyme A to the primary amine of a wide range of acceptor substrates.

The function of the putative protein disclosed in NM_001001985.3 was thus unknown.

As shown in the Examples, the inventors provide the evidence that the protein having for sequence SEQ ID NO: 2 is the aspartate-N-acetyltransferase that catalyses NAA synthesis in brain.

Another object of the invention is an isolated rat aspartate-N-acetyltransferase having for sequence SEQ ID NO: 3 or an amino acid sequence having at least 90% identity with SEQ ID NO: 3, preferably at least 91%, 92%, 93%, 94%, 95%, 96%, 97%, 98% and more preferably at least 99% identity with SEQ ID NO: 3.

SEQ ID NO: 3 is disclosed under XM_223538.4 NCBI accession number and the function of the putative protein disclosed in XM_223538.4 was unknown.

Another object of the invention is a nucleic acid sequence encoding SEQ ID NO : 1, SEQ ID NO: 2 or SEQ ID NO: 3 or an amino acid sequence having at least 90%, 95%, 96%, 97%, 98%, 99% identity with SEQ ID NO: 1, SEQ ID NO: 2 or SEQ ID NO: 3.

According to the invention, a nucleic acid sequence encoding SEQ ID NO: 1, SEQ ID NO: 2 or SEQ ID NO: 3 can be determined using standard molecular biology techniques known by the person skilled in the art (as described in Sambrook et al, eds, Molecular Cloning: A laboratory manual, 2^{nd} edition, Cold Spring Harbor, NY 1989) and the sequence information provided herein.

In one embodiment, a nucleic acid sequence encoding SEQ ID NO: 1 has for sequence SEQ ID NO 4.

In another embodiment, a nucleic acid sequence encoding SEQ ID NO: 2 has for sequence SEQ ID NO: 5.

In another embodiment, a nucleic acid sequence encoding SEQ ID NO: 3 has for sequence SEQ ID NO: 6.

Another object of the invention is a fragment of the nucleic acid sequence encoding SEQ ID NO: 1, SEQ ID NO: 2 or SEQ ID NO: 3, said fragment being suitable for use as probe or primer for the detection of aspartate-N-acetyltransferase gene, cDNA or mRNA in a sample.

In one embodiment, said fragment comprises a region of the nucleotide sequence encoding SEQ ID NO: 1, SEQ ID NO: 2 or SEQ ID NO: 3, that hybridizes under stringent conditions to at least about 12, preferably about 25, more preferably about 50, 75, 100, 125, 150, 175, 200, 250, 300, 350 or 400 consecutive nucleotides of the sense or anti-sense nucleic acid sequence encoding SEQ ID NO:1, SEQ ID NO:2 or SEQ ID NO: 3.

According to the invention, the probes or primers may comprise a label group attached thereto such as a radioisotope, a fluorescent compound, an enzyme or an enzyme co-factor. Said probes and primers may be used as part of a diagnostic test kit for identifying cells or tissues which express or not the aspartate-N-acetyltransferase enzyme, by measuring for example mRNA or cDNA levels or by determining whether the gene encoding the aspartate-N-acetyltransferase enzyme has been mutated or deleted.

Another object of the invention is a vector comprising a nucleic acid sequence encoding SEQ ID NO: 1, SEQ ID NO: 2 or SEQ ID NO: 3 or an amino acid sequence having at least 90%, 95%, 96%, 97%, 98%, 99% identity with SEQ ID NO: 1, SEQ ID NO: 2 or SEQ ID NO: 3 or a fragment thereof.

As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the invention is intended to include such other forms of expression vectors, such as viral vectors (e.g., replication defective retroviruses, adenoviruses and adeno-associated viruses), which serve equivalent functions.

The recombinant expression vectors of the invention comprise a nucleic acid of the invention in a form suitable for expression of the nucleic acid in a host cell. This means that the recombinant expression vectors include one or more regulatory sequences, selected on the basis of the host cells to be used for expression, which is operably linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an in vitro transcription/translation system or in a host cell when the vector is introduced into the host cell).

The term "regulatory sequence" is intended to include promoters, enhancers and other expression control elements (e.g., polyadenylation signals). Such regulatory sequences are described, for example, in Goeddel, Gene Expression Technology: Methods in Enzymology 185, Academic Press, San Diego, Calif. (1990). Regulatory sequences include those which direct constitutive expression of a nucleotide sequence in many types of host cell and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein.

The recombinant expression vectors of the invention can be designed for expression of a polypeptide of the invention in prokaryotic (e.g., E. coli) or eukaryotic cells (e.g., insect cells (using baculovirus expression vectors), yeast cells or mammalian cells). Suitable host cells are discussed further in Goeddel, supra. Alternatively, the recombinant expression vector can be transcribed and translated in vitro, for example using T7 promoter regulatory sequences and T7 polymerase.

Expression of proteins in prokaryotes is most often carried out in E. coli with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson (1988) Gene 67:31-40), pMAL (New England Biolabs, Beverly, Mass.) and pRIT5 (Pharmacia, Piscataway, N.J.) which fuse glutathione S-transferase (GST), maltose E binding protein, or protein A, respectively, to the target recombinant protein.

Examples of suitable inducible non-fusion E. coli expression vectors include pTrc (Amann et al., (1988) Gene 69:301-315) and pET 11d (Studier et al., Gene Expression Technology: Methods in Enzymology 185, Academic Press, San Diego, Calif. (1990) 60-89). Target gene expression from the pTrc vector relies on host RNA polymerase transcription from a hybrid trp-lac fusion promoter. Target gene expression from the pET 1d vector relies on transcription from a T7 gn10-lac fusion promoter mediated by a coexpressed viral RNA polymerase (T7 gn1). This viral polymerase is supplied by host strains BL21(DE3) or HMS174(DE3) from a resident ) prophage harboring a T7 gn1 gene under the transcriptional control of the lacUV 5 promoter.

Examples of plasmids include replicating plasmids comprising an origin of replication, or integrative plasmids, such as pUC, pcDNA, pBR and the like.

Examples of viral vector include adenoviral, retroviral, herpes virus or AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells...

Another object of the invention is a host cell comprising a nucleic acid sequence encoding SEQ ID NO: 1, SEQ ID NO: 2 or SEQ ID NO: 3 or an amino acid sequence having at least 95%, 96%, 97%, 98%, 99% identity with SEQ ID NO: 1, SEQ ID NO: 2 or SEQ ID NO: 3 or a vector as described here above.

A host cell can be any prokaryotic (e.g., E. coli) or eukaryotic cell (e.g., insect cells, yeast or mammalian cells).

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid into a host cell, including calcium phosphate or calcium chloride coprecipitation, DEAE-dextran-mediated transfection, lipofection, or electroporation. Suitable methods for transforming or transfecting host cells can be found in Sambrook, et al. (supra), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., for resistance to antibiotics) is generally introduced into the host cells along with the gene of interest. Preferred selectable markers include those which confer resistance to drugs, such as G418, hygromycin and methotrexate. Cells stably transfected with the introduced nucleic acid can be identified by drug selection (e.g., cells that have incorporated the selectable marker gene will survive, while the other cells die).

In a particular embodiment of the invention, the host cell comprises a mutated nucleic acid sequence encoding SEQ ID NO: 1, wherein said mutation leads to an alteration (diminution) of the enzymatic activity of aspartate-N-actelytransferase.

One example of such mutation is R220K or D168E.

The host cells of the invention can also be used to produce non-human transgenic animals. For example, in one embodiment, a host cell of the invention is a fertilized oocyte or an embryonic stem cell into which a sequence encoding a polypeptide of the invention has been introduced. Such host cells can then be used to create non-human transgenic animals in which exogenous sequences encoding a polypeptide of the invention have been introduced into their genome or homologous recombinant animals in which endogenous encoding a polypeptide of the invention sequences have been altered. Such animals are useful for studying the function and/or activity of the polypeptide and for identifying and/or evaluating modulators of polypeptide activity.

As used herein, a "transgenic animal" is a non-human animal, preferably a mammal, more preferably a rodent such as a rat or mouse, in which one or more of the cells of the animal includes a transgene.

Other examples of transgenic animals include non-human primates, sheep, dogs, cows, goats, chickens, amphibians, etc. A transgene is exogenous DNA which is integrated into the genome of a cell from which a transgenic animal develops and which remains in the genome of the mature animal, thereby directing the expression of an encoded gene product in one or more cell types or tissues of the transgenic animal.

As used herein, a "homologous recombinant animal" is a non-human animal, preferably a mammal, more preferably a mouse, in which an endogenous gene has been altered by homologous recombination between the endogenous gene and an exogenous DNA molecule introduced into a cell of the animal, e.g., an embryonic cell of the animal, prior to development of the animal.

A transgenic animal of the invention can be created by introducing nucleic acid encoding a polypeptide of the invention into the male pronuclei of a fertilized oocyte, e.g., by microinjection, retroviral infection, and allowing the oocyte to develop in a pseudopregnant female foster animal. Intronic sequences and polyadenylation signals can also be included in the transgene to increase the efficiency of expression of the transgene. A tissue-specific regulatory sequence(s) can be operably linked to the transgene to direct expression of the polypeptide of the invention to particular cells. Methods for generating transgenic animals via embryo manipulation and microinjection, particularly animals such as mice, have become conventional in the art and are described, for example, in U.S. Pat. Nos. 4,736,866 and 4,870,009, 4,873,191 and in Hogan, Manipulating the Mouse Embryo, (Cold Spring Harbor Laboratory Press, Cold Spring Harbor, N.Y., 1986). Similar methods are used for production of other transgenic animals. A transgenic founder animal can be identified based upon the presence of the transgene in its genome and/or expression of mRNA encoding the transgene in tissues or cells of the animals. A transgenic founder animal can then be used to breed additional animals carrying the transgene. Moreover, transgenic animals carrying the transgene can further be bred to other transgenic animals carrying other transgenes.

To create a homologous recombinant animal, a vector is prepared which contains at least a portion of a gene encoding a polypeptide of the invention into which a deletion, addition or substitution has been introduced to thereby alter, e.g., functionally disrupt, the gene.

In a preferred embodiment, the vector is designed such that, upon homologous recombination, the endogenous gene is functionally disrupted (i.e., no longer encodes a functional protein; also referred to as a "knock out" vector).

Alternatively, the vector can be designed such that, upon homologous recombination, the endogenous gene is mutated or otherwise altered but still encodes functional protein (e.g., the upstream regulatory region can be altered to thereby alter the expression of the endogenous protein).

In the homologous recombination vector, the altered portion of the gene is flanked at its 5' and 3' ends by additional nucleic acid of the gene to allow for homologous recombination to occur between the exogenous gene carried by the vector and an endogenous gene in an embryonic stem cell. The additional flanking nucleic acid sequences are of sufficient length for successful homologous recombination with the endogenous gene. Typically, several kilobases of flanking DNA (both at the 5' and 3' ends) are included in the vector (see, e.g., Thomas and Capecchi (1987) Cell 51:503 for a description of homologous recombination vectors). The vector is introduced into an embryonic stem cell line (e.g., by electroporation) and cells in which the introduced gene has homologously recombined with the endogenous gene are selected (see, e.g., Li et al. (1992) Cell 69:915). The selected cells are then injected into a blastocyst of an animal (e.g., a mouse) to form aggregation chimeras (see, e.g., Bradley in Teratocarcinomas and Embryonic Stem Cells: A Practical Approach, Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley (1991) Current Opinion in Biotechnology 2:823-829 and in PCT Publication Nos. WO 90/11354, WO 91/01140, WO 92/0968 and WO 93/04169.

Another object of the invention is thus a non-human transgenic animal for the aspartate-N-acetyltransferase.

In one embodiment, said non-human transgenic animal is knock-out for the aspartate-N-acetyltransferase. In another embodiment, said non-human transgenic animal expresses an altered aspartate-N-acetyltransferase. In another embodiment, said non-human transgenic animal over-expresses aspartate-N-acetyltransferase.

Another object of the invention is an antibody or fragment thereof that specifically recognises aspartate-N-acetyltransferase having for sequence SEQ ID NO: 1, SEQ ID NO: 2 or SEQ ID NO: 3 or an amino acid sequence having at least 95%, 96%, 97%, 98%, 99% identity with SEQ ID NO: 1, SEQ ID NO: 2 or SEQ ID NO: 3.

According to the invention, the terms "antibody" refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (λ) and kappa (κ). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non hypervariable or framework regions (FR) influence the overall domain structure and hence the combining site. Complementarity determining regions (CDRs) refer to amino acid sequences which, together, define the binding affinity and specificity of the natural Fv region of a native immunoglobulin binding-site. The light and heavy chains of an immunoglobulin each have three CDRs, designated L-CDR1, L-CDR2, L-CDR3 and H-CDR1, H-CDR2, H-CDR3, respectively. Therefore, an antigen-binding site includes six CDRs, comprising the CDR set from each of a heavy and a light chain V region. Framework Regions (FRs) refer to amino acid sequences interposed between CDRs, i.e. to those portions of immunoglobulin light and heavy chain variable regions that are relatively conserved among different immunoglobulins in a single species, as defined by Kabat, et al (Sequences of Proteins of Immunological Interest (National Institutes of Health, Bethesda, Md., 1991). As used herein, a "human framework region" is a framework region that is substantially identical (about 85%, or more, in particular 90%, 95%, or 100%) to the framework region of a naturally occurring human antibody.

The term "monoclonal antibody" or "mAb" as used herein refers to an antibody molecule of a single amino acid composition, that is directed against a specific antigen and that is produced by a single clone of B cells or hybridoma.

The term "chimeric antibody" refers to an engineered antibody which comprises a VH domain and a VL domain of an antibody derived from a non-human animal, in association with a CH domain and a CL domain of another antibody, in particular a human antibody. As the non-human animal, any animal such as mouse, rat, hamster, rabbit or the like can be used.

The term "humanized antibody" refers to antibodies in which the framework or "complementarity determining regions" (CDR) have been modified to comprise the CDR from a donor immunoglobulin of different specificity as compared to that of the parent immunoglobulin. In a preferred embodiment, a mouse CDR is grafted into the framework region of a human antibody to prepare the "humanized antibody".

"Antibody fragments" comprise a portion of an intact antibody, preferably the antigen binding or variable region of the intact antibody. Examples of antibody fragments include Fv, Fab, F(ab')₂, Fab', dsFv, scFv, sc(Fv)₂, diabodies and multispecific antibodies formed from antibody fragments.

In one embodiment of the invention, said antibody may be a human, non-human, chimeric and/or humanized antibody. Such non-human antibody may be goat, mouse, sheep, horse, chicken, rabbit, or rat antibody. In addition, the antibody of the invention may be polyclonal or monoclonal.

Antibodies according to invention may be produced by any technique known in the art, such as, without limitation, any chemical, biological, genetic or enzymatic technique, either alone or in combination.

Polyclonal antibodies can be prepared by immunizing a suitable subject with a aspartate-N-acetyltransferase polypeptide of the invention as an immunogen. The antibody titer in the immunized subject can be monitored over time by standard techniques, such as with an enzyme linked immunosorbent assay (ELISA) using immobilized polypeptide. If desired, the antibody molecules can be isolated from the mammal (e.g., from the blood) and further purified by well-known techniques, such as protein A chromatography to obtain the IgG fraction. Alternatively, antibodies specific for a protein or polypeptide of the invention can be selected for (e.g., partially purified) or purified by, e.g., affinity chromatography. At an appropriate time after immunization, e.g., when the specific antibody titers are highest, antibody-producing cells can be obtained from the subject and used to prepare monoclonal antibodies by standard techniques, such as the hybridoma technique originally described by Kohler and Milstein (1975) Nature 256:495-497, the human B cell hybridoma technique (Kozbor et al. (1983) Immunol. Today 4:72), the EBV-hybridoma technique (Cole et al. (1985), Monoclonal Antibodies and Cancer Therapy, Alan R. Liss, Inc., pp. 77-96) or trioma techniques. The technology for producing hybridomas is well known (see generally Current Protocols in Immunology (1994) Coligan et al. (eds.) John Wiley & Sons, Inc., New York, N.Y.). Hybridoma cells producing a monoclonal antibody of the invention are detected by screening the hybridoma culture supernatants for antibodies that bind the polypeptide of interest, e.g., using a standard ELISA assay. Alternative to preparing monoclonal antibody-secreting hybridomas, a monoclonal antibody directed against a polypeptide of the invention can be identified and isolated by screening a recombinant combinatorial immunoglobulin library (e.g., an antibody phage display library) with the polypeptide of interest. Kits for generating and screening phage display libraries are commercially available (e.g., the Pharmacia Recombinant Phage Antibody System, Catalog No. 27-9400-01; and the Stratagene SurfZAP(TM) Phage Display Kit, Catalog No. 240612). Additionally, examples of methods and reagents particularly amenable for use in generating and screening antibody display library can be found in, for example, U.S. Pat. No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al. (1991) Bio/Technology 9:1370-1372; Hay et al. (1992) Hum. Antibod. Hybridomas 3:81-85; Huse et al. (1989) Science 246:1275-1281; Griffiths et al. (1993) EMBO J. 12:725-734.

The antibody directed against aspartate-N-acetyltransferase can be used to isolate the polypeptide by standard techniques, such as affinity chromatography or immunoprecipitation. Moreover, such an antibody can be used to detect the protein (e.g., in a cellular lysate or cell supernatant) in order to evaluate the abundance and pattern of expression of the polypeptide. The antibodies can also be used diagnostically to monitor protein levels in tissue as part of a clinical testing procedure, e.g., to, for example, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

In a particular embodiment, said antibody specifically recognises one of the following antigenic peptide: (C)NTAFRGLRGHPRAGLL (human sequence) (C)NTAFRGLRQHPRTQLL (murine sequence) (P44) and (C)MSVDSRFRGKGIAKALG (P45) (murine and human sequence).

Another object of the invention is a method for screening or identifying compounds capable of modulating the aspartate-N-acetyltransferase enzyme activity, comprising
- contacting a compound to be tested with a model system expressing the aspartate-N-acetyltransferase enzyme having for sequence SEQ ID NO: 1, SEQ ID NO: 2 or SEQ ID NO: 3 in conditions allowing the enzymatic reaction, and
- determining the aspartate-N-acetyltransferase enzyme activity.

In one embodiment, said compounds are stimulating the aspartate-N-acetyltransferase enzyme activity.

In another embodiment, said compounds are inhibiting the aspartate-N-acetyltransferase enzyme activity.

The test compounds may be obtained using any of the numerous approaches in combinatorial library methods known in the art, including: biological libraries, spatially addressable parallel solid phase or solution phase libraries, synthetic libraries methods requiring deconvolution or synthetic library methods using affinity chromatography selection.

In one embodiment of the invention, the model system expressing the aspartate-N-acetyltransferase enzyme is extracts of cells transfected with the sequence SEQ ID NO: 4, or the nucleic acid sequence encoding SEQ ID NO: 2 or SEQ ID NO: 3 or washed membranes prepared from such extracts.

In this embodiment, this screening assay is based on the aspartate dependent conversion of acetylCoA to CoASH. CoASH can react with DTNB to form a chromogen absorbing at 412 nm.

In an example of said screening assay, a reaction mixture comprising Hepes (25 mM, pH 7.1), potassium phosphate (20 mM), MgC12 (1 mM), acetylCoA (200 µM) and L-aspartate (0-1 mM), is distributed into a multiwells plate. The compound to be tested is added into the well at a desired range concentration. The assay is then started by addition of the cell extracts or cell membranes. DTNB (5 mM) is added after a suitable time (about 1 hour) at 30°C. As DTNB inhibits the aspartate-N-acetyltransferase activity at high concentration, it stops the reaction and allows the formation of chromogen. The plate is finally read at 412 nm.

The formation of chromogen is therefore representative of the aspartate-N-acetyltransferase activity and thus is indicative of the modulation of the enzyme induced by the compound to be tested.

In another example of said screening assay, the aspartate-N-acetyltransferase activity can be assayed radiochemically by contacting cell extracts with a reaction mixture comprising [¹⁴C]L-aspartate as described in the Examples.

NAA is also known to exert a retro-inhibition on the aspartate-N-acetyltransferase activity. Thus, addition of a suitable concentration of NAA (for example 1 to 20 mM) at the same time than the compound to be tested allows the screening of compounds that antagonise the retro-inhibition of NAA.

In another embodiment of the invention, the model system expressing the aspartate-N-acetyltransferase enzyme is cells transfected with the sequence SEQ ID NO: 4, or the nucleic acid sequence encoding SEQ ID NO: 2 or SEQ ID NO: 3.

In this embodiment, cells, such as HEK293T cells, transfected with SEQ ID NO: 4, or the nucleic acid sequence encoding SEQ ID NO: 2 or SEQ ID NO: 3 are cultured for 48 hours in the presence or absence of the compound to be tested.

The concentration of NAA in the medium or intracellularly can then be quantified by isotope dilution gas chromatography-mass spectrometry as described in Kelley and Stamas (21) and is representative of the aspartate-N-acetyltransferase activity.

Another object of the invention is a method for determining whether a subject has or is at risk of having an aspartate-N-acetyltransferase enzyme-linked disease, comprising detecting, in a sample obtained from said subject, genetic lesions or mutations in the aspartate-N-acetyltransferase gene.

In one embodiment of the invention, said aspartate-N-acetyltransferase enzyme activity-linked disease is hypoacetylaspartia (aspartate N-acetyltransferase deficiency), epilepsy, leukodystrophies, Alzheimer's disease, multiple sclerosis, amyotrophic lateral sclerosis, stroke, AIDS encephalopathy or herpes encephalitis.

In another embodiment of the invention, said aspartate-N-acetyltransferase enzyme activity-linked disease is a disease associated with aberrant expression or activity of aspartate-N-acetyltransferase.

As used herein, the term "sample" means any biological sample derived from a patient. Examples of such samples include fluids, tissues, cell samples, organs, biopsies, etc. Preferred biological samples are whole blood, serum, or plasma.

In one embodiment of the invention, the method as described here above includes detecting, in a sample obtained from the subject, the presence or absence of a genetic lesion or mutation characterized by at least one of an alteration affecting the integrity of a gene encoding aspartate-N-acetyltransferase, or the mis-expression of the gene encoding aspartate-N-acetyltransferase.

For example, such genetic lesions or mutations can be detected by ascertaining the existence of at least one of: 1) a deletion of one or more nucleotides from the gene; 2) an addition of one or more nucleotides to the gene; 3) a substitution of one or more nucleotides of the gene; 4) a chromosomal rearrangement of the gene; 5) an alteration in the level of a messenger RNA transcript of the gene; 6) an aberrant modification of the gene, such as of the methylation pattern of the genomic DNA; 7) the presence of a non-wild type splicing pattern of a messenger RNA transcript of the gene; 8) a non-wild type level of the protein encoded by the gene; 9) an allelic loss of the gene; and 10) an inappropriate post-translational modification of the protein encoded by the gene. As described herein, there are a large number of assay techniques known in the art which can be used for detecting lesions in a gene.

In certain embodiments, detection of the lesion involves the use of a probe/primer in a polymerase chain reaction (PCR) (see, e.g., U.S. Pat. Nos. 4,683,195 and 4,683,202), such as anchor PCR or RACE PCR, or, alternatively, in a ligation chain reaction (LCR) (see, e.g., Landegran et al. (1988) Science 241:1077-1080; and Nakazawa et al. (1994) Proc. Natl. Acad. Sci. USA 91:360-364), the latter of which can be particularly useful for detecting point mutations in a gene (see, e.g., Abravaya et al. (1995) Nucleic Acids Res. 23:675-682). This method can include the steps of collecting a sample of cells from a patient, isolating nucleic acid (e.g., genomic, mRNA or both) from the cells of the sample, contacting the nucleic acid sample with one or more primers which specifically hybridize to the selected gene under conditions such that hybridization and amplification of the gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

In an alternative embodiment, mutations in a selected gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA.

In yet another embodiment, any of a variety of sequencing reactions known in the art can be used to directly sequence the selected gene and detect mutations by comparing the sequence of the sample nucleic acids with the corresponding wild-type (control) sequence. Examples of sequencing reactions include those based on techniques developed by Maxam and Gilbert ((1977) Proc. Natl. Acad. Sci. USA 74:560) or Sanger ((1977) Proc. Natl. Acad. Sci. USA 74:5463). It is also contemplated that any of a variety of automated sequencing procedures can be utilized when performing the diagnostic assays ((1995) Bio/Techniques 19:448), including sequencing by mass spectrometry (see, e.g., PCT Publication No. WO 94/16101; Cohen et al. (1996) Adv. Chromatogr. 36:127-162; and Griffin et al. (1993) Appl. Biochem. Biotechnol. 38:147-159).

In other embodiments, alterations in electrophoretic mobility will be used to identify mutations in genes. For example, single strand conformation polymorphism (SSCP) may be used to detect differences in electrophoretic mobility between mutant and wild type nucleic acids (Orita et al. (1989) Proc. Natl. Acad. Sci. USA 86:2766; see also Cotton (1993) Mutat. Res. 285:125-144; Hayashi (1992) Genet. Anal. Tech. Appl. 9:73-79). Single-stranded DNA fragments of sample and control nucleic acids will be denatured and allowed to renature. The secondary structure of single-stranded nucleic acids varies according to sequence, and the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet. 7:5).

In yet another embodiment, the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a 'GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys. Chem. 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163); Saiki et al. (1989) Proc. Natl. Acad. Sci. USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3' end of one primer where, under appropriate conditions, mismatch can prevent or reduce polymerase extension (Prossner (1993) Tibtech 11:238). In addition, it may be desirable to introduce a novel restriction site in the region of the mutation to create cleavage-based detection (Gasparini et al. (1992) Mol. Cell Probes 6: 1). It is anticipated that in certain embodiments amplification may also be performed using Taq ligase for amplification (Barany (1991) Proc. Natl. Acad. Sci. USA 88:189). In such cases, ligation will occur only if there is a perfect match at the 3' end of the 5' sequence making it possible to detect the presence of a known mutation at a specific site by looking for the presence or absence of amplification.

A preferred agent for amplifying the aspartate-N-acetyletransferase gene is a nucleic acid primer capable of hybridizing to genomic DNA encoding aspartate-N-acetyltransferase.

The nucleic acid primer can be a fragment of the nucleic acid sequence encoding aspartate-N-acetyltransferase such as an oligonucleotide of at least 10, 15, 30 nucleotides in length and sufficient to specifically hybridize under stringent conditions to genomic DNA encoding aspartate-N-acetyltransferase.

In a particular embodiment of the invention, at least 3 couples of primers are used to amplify the 3 exons of the gene encoding aspartate-N-acetyltransferase. Examples of said 3 couples of primers are shown in the following table:

| Mutation search | Sense primer | Antisense primer |
|---|---|---|
| (exon 1) | TTGCCCTGGGCCCGGCCGT | CACGCCCATAGCCAGCCCTGC |
| | (SEQ ID NO : 7) | (SEQ ID NO : 8) |
| (exon 2) | GTCCCTAGCGGGCTTCGCC | TGCCCTGAGGTCGCTGGAGTG |
| | (SEQ ID NO :9) | (SEQ ID NO : 10) |
| (exon 3) | TGTCTCACCCCAGAGGTGGAG | CCAATTGAGCGTCTGAAAGCAGG |
| | (SEQ ID NO : 11) | (SEQ ID NO : 12) |
| | | |

As exon 1 of the aspartate-N-acetyltransferase gene is highly GC rich, the inventors also designed two couples of primers allowing a better amplification of exon 1.

Said couples of primers are shown in the following table:

| Mutation search | Sense primer | Antisense primer |
|---|---|---|
| (exon 1 ; 5' part) | TTGCCCTGGGCCCGGCCGT | AGGCCGTGTTAGGGATGCGCTC |
| | (SEQ ID NO : 7) | (SEQ ID NO : 13) |
| (exon 1 ; 3' part) | TTGCCCTGGGCCCGGCCGT | CACGCCCATAGCCAGCCCTGC |
| | (SEQ ID NO : 14) | (SEQ ID NO : 8) |

In another embodiment of the invention, the method for determining whether a subject has or is at risk of having an aspartate-N-acetyltransferase enzyme-linked disease, that comprises detecting genetic lesions or mutations in the aspartate-N-acetyltransferase gene, may further comprise an additional step of validating whether the genetic lesion or mutation has an effect on aspartate-N-acetyltransferase activity.

This validation step may be carried out by transfecting the nucleic acid sequence sequenced previously in cells such as HEK293T cells as shown in the Examples.

The aspartate-N-acetyltransferase activity can then be determined using the assays as described here above.

In particular, the aspartate-N-acetyltransferase activity can be determined using an assay based on the aspartate dependent conversion of acetylCoA to CoASH. CoASH can react with DTNB to form a chromogen absorbing at 412 nm.

In an example of said aspartate-N-acetyltransferase activity assay, a reaction mixture comprising Hepes (25 mM, pH 7.1), potassium phosphate (20 mM), MgC12 (1 mM), acetylCoA (200 µM) and L-aspartate (0-1 mM), is distributed into a multiwells plate. The compound to be tested is added into the wells at a desired range concentration. The assay is then started by addition of the cell extracts or cell membranes. DTNB (5 mM) is added after a suitable time (about 1 hour) at 30°C. As DTNB inhibits the aspartate-N-acetyltransferase activity at high concentration, it stops the reaction and allows the formation of chromogen. The plate is finally read at 412 nm.

The formation of chromogen is therefore representative of the aspartate-N-acetyltransferase activity and thus is indicative of the activity of the enzyme.

In another example of aspartate-N-acetyltransferase activity assay, the aspartate-N-acetyltransferase activity can be assayed radiochemically by contacting cell extracts with a reaction mixture comprising [¹⁴C]L-aspartate as described in the Examples.

The determined aspartate-N-acetyltransferase activity of the previously sequenced sequence is finally compared to the aspartate-N-acetyltransferase activity obtained in similar assay wherein cells were transfected with SEQ ID NO: 4.

This assay thus allows determining whether the previously sequenced sequence has an aspartate-N-acetyltransferase activity that is similar or altered compared to the activity of the wild enzyme.

Another means to determine whether the previously sequenced sequence has an aspartate-N-acetyltransferase activity is to check the expression of the protein and compare it to the expression of the wild protein.

This assay can be carried out by western-blotting the proteins from cell extracts using antibodies against a tag added at the end of the sequence. Such an assay is described in the Examples. Briefly, C- or N-terminal tagged proteins (with a polyHis and/or a myc tag) are prepared by inserting SEQ ID NO: 4 or the previously sequenced sequence in pEF6MycHisA (C-tagged protein) or in pEF6HisB (N-tagged protein). Cells, such as HEK293T cells, are transfected with said expression vectors. After 48h of culture, cells extracts are prepared and western-blots are performed with a pentaHIS antibody.

Another object of the invention is a kit for amplifying the nucleic acid sequence of the aspartate-N-acetyltransferase in a biological sample (a test sample). Such kit can be used to determine if a subject has or is at risk of having an aspartate-N-acetyltransferase enzyme-linked disease.

The kit, for example, can comprise a labelled or non-labelled compound or agent capable of detecting mRNA or DNA encoding aspartate-N-acetyltransferase in a biological sample. The kit can comprise, for example: (1) an oligonucleotide, e.g., a detectably labelled oligonucleotide, which hybridizes to a nucleic acid sequence encoding aspartate-N-acetyltransferase or (2) at least one couple of primers useful for amplifying a nucleic acid molecule encoding aspartate-N-acetyltransferase.

The kit can also comprise, e.g., a buffering agent, a preservative, or a protein stabilizing agent. The kit can also comprise components necessary for detecting the detectable agent (e.g., an enzyme or a substrate). The kit can also contain a control sample or a series of control samples which can be assayed and compared to the test sample contained. Each component of the kit is usually enclosed within an individual container.

In a particular embodiment of the invention, the kit comprises at least three couples of primers that allow the amplification of each of the three exons of the aspartate-N-acetyletransferase.

According to the invention, exon 1 starts at the ATG (position 1 in SEQ ID NO: 4) and ends at the CCG (position 365-367); exon 2 starts at the CCC (position 368-370) and ends at the CTG (position 530-532); and exon 3 starts at the GTT (position 533-535) and ends at the TGA (position 898-900).

In one embodiment, said kit comprises the following couples of primers:

| Mutation search | Sense primer | Antisense primer |
|---|---|---|
| (exon 1) | TTGCCCTGGGCCCGGCCGT | CACGCCCATAGCCAGCCCTGC |
| | (SEQ ID NO : 7) | (SEQ ID NO : 8) |
| (exon 2) | GTCCCTAGCGGGCTTCGCC | TGCCCTGAGGTCGCTGGAGTG |
| | (SEQ ID NO : 9) | (SEQ ID NO : 10) |
| (exon 3) | TGTCTCACCCCAGAGGTGGAG | CCAATTGAGCGTCTGAAAGCAGG |
| | (SEQ ID NO : 11) | (SEQ ID NO : 12) |

In another embodiment, said kit comprises the following couples of primers:

| Mutation search | Sense primer | Antisense primer |
|---|---|---|
| (exon 1 ; 5' part) | TTGCCCTGGGCCCGGCCGT | AGGCCGTGTTAGGGATGCGCTC |
| | (SEQ ID NO : 7) | (SEQ ID NO : 13) |
| (exon 1 ; 3' part) | TTGCCCTGGGCCCGGCCGT | CACGCCCATAGCCAGCCCTGC |
| | (SEQ ID NO : 14) | (SEQ ID NO : 8) |
| (exon 2) | GTCCCTAGCGGGCTTCGCC | TGCCCTGAGGTCGCTGGAGTG |
| | (SEQ ID NO : 9) | (SEQ ID NO : 10) |
| (exon 3) | TGTCTCACCCCAGAGGTGGAG | CCAATTGAGCGTCTGAAAGCAGG |
| | (SEQ ID NO : 11) | (SEQ ID NO : 12) |

Another object of the invention is a method for treating an aspartate-N-acetyltransferase-linked disease wherein the level of NAA is decreased, comprising administering to a subject in need thereof a therapeutically effective amount of aspartate.

Another object of the invention is aspartate for treating or for use in treating aspartate-N-acetyltransferase linked disease wherein the level of NAA is decreased.

In one embodiment of the invention, said aspartate-N-acetyltransferase-linked disease wherein the level of NAA is decreased is epilepsy, Alzheimer's disease, AIDS encephalopathy, herpes encephalopathy, stroke, leucodystrophy.

The NAA decrease may indeed be part of a vicious circle leading to a reinforcement of the pathological condition. By increasing the level of NAA, the vicious circle would be interrupted. Without willing to be bound to a theory, the inventors state that administrating aspartate to a subject in need thereof would enhance the aspartate-N-acetyltrasnferase activity and thus restore a normal level of NAA.

Another object of the invention is a method for treating an aspartate-N-acetyltransferase-linked disease wherein no NAA is produced, comprising administering to a subject in need thereof a therapeutically effective amount of NAA or precursors thereof.

Another object of the invention is NAA or precursors thereof for treating or for use in treating aspartate-N-acetyltransferase linked disease wherein no NAA is produced.

In one embodiment of the invention, said aspartate-N-acetyltransferase-linked disease wherein no NAA is produced is hypoacetylaspartia.

By injecting NAA or precursors thereof, the NAA level may be increased in neurons and be also provided to other cells of the CNS, as, e.g. oligodendrocytes, which use NAA to form myelin.

Examples of precursors of NAA include, but are not limited to, N-acetylaspartate monomethyl ester and N-acetylaspartate dimethyl ester.

In the context of the invention, the term "treating" or "treatment", as used herein, means reversing, alleviating, inhibiting the progress of, or preventing the disorder or condition to which such term applies, or one or more symptoms of such disorder or condition.

As used herein, the term "subject" or "subject in need thereof", refers to a human or non-human mammal affected or likely to be affected with an aspartate-N-acetyltransferase-linked disease.

By a "therapeutically effective amount" of the polypeptide or nucleic acid molecule of the invention is meant a sufficient amount of the nucleic acid molecule or polypeptide to treat aspartate-N-acetyltransferase linked disease at a reasonable benefit/risk ratio applicable to any medical treatment. It will be understood, however, that the total daily usage of the polypeptides or nucleic acid molecules and compositions of the present invention will be decided by the attending physician within the scope of sound medical judgment. The specific therapeutically effective dose level for any particular patient will depend upon a variety of factors including the disorder being treated and the severity of the disorder; activity of the specific polypeptide employed; the specific composition employed, the age, body weight, general health, sex and diet of the patient; the time of administration, route of administration, and rate of excretion of the specific polypeptide employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved.

Aspartate or NAA may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The invention will further be illustrated in view of the following figure and example.

### BRIEF DESCRIPTION OF THE FIGURES

**Figure 1****. Multiple alignment of NAT8L sequences.** The alignment shows the sequences from *Homo sapiens* (Hsap; NCBI accession nr EAW82541), *Mus musculus* (Mmus; NP_001001985) and *Danio rerio* (Drer; NP_001077308). Strictly conserved residues are indicated in bold. A 30 residue hydrophobic region is underlined in the human sequence.
**Figure 2****. Effect of transfection of HEK cells with mouse NAT8L or NAT14 on the NAA concentration in the medium and the Asp-NAT activity in cell extracts.**
   Cells were transfected with plasmids expressing NAT8L or NAT14, either as such (WT) or as C-terminal (CT) or N-terminal (NT) polyHis tag fusion proteins. Transfection with an empty plasmid (EP) was used as a control. After 48 h, the NAA concentration was measured by GC/MS in the medium (*B*) and the Asp-NAT activity was assayed in cell extracts (*C*). The indicated extracts were also analysed by Western blotting with an anti-polyHis tag antibody to evaluate the level of expression of the recombinant proteins (*A*). These data are representative (*A*) or the means ± SEM (*B, C*) of three independent transfection experiments.

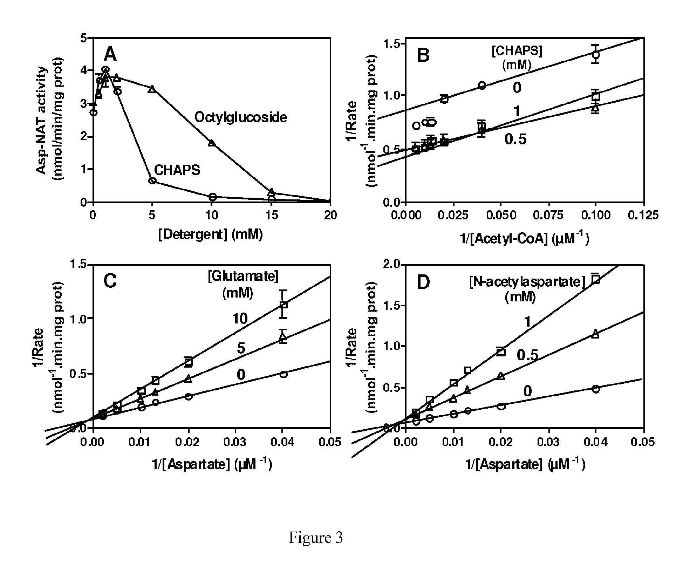

**Figure 3****. Kinetic properties of the Asp-NAT activity of NAT8L.**
   HEK cells were transfected with untagged mouse NAT8L and the enzymatic activity was determined radiochemically on washed membrane pellets as described in the Materials and Methods. (*A*) Assay medium contained 50 µM L-aspartate, 200 µM acetyl-CoA, and the indicated concentrations of CHAPS or octylglucoside. (*B*) Assay medium contained 20 µM aspartate and the indicated concentrations of acetyl-CoA and CHAPS. (*C*) and (*D*) : assay medium contained 200 µM acetyl-CoA, 0.5 mM CHAPS and variable concentrations of aspartate. Panels (*B*), (*C*) and (*D*) show double reciprocal plots. The results shown are the means ± SEM for three determinations performed with extracts derived from independent transfections. In some cases, the SEM is not visible because it is smaller than the size of the symbol.

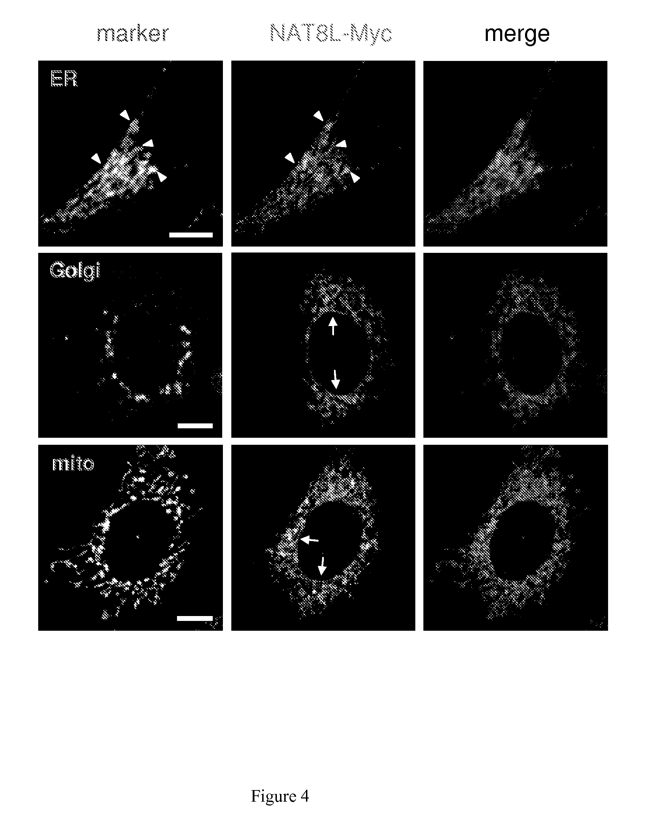

**Figure 4****. Co-localization of *myc*-tagged NAT8L with the endoplasmic reticulum (ER) and complete segregation from the Golgi complex and mitochondria (mito).**
   CHO cells were transfected to express low levels of C-terminal *myc*-tagged NAT8L and immunolabelled with an antibody against the *myc*-antigen (central column, green in merge at right). Upper panel: double immunolabelling for KDEL-bearing proteins to label the ER (left, red in merge). Middle panel: cells were co-transfected with the PDIb-HA construct to immunolabel the Golgi apparatus (HA; left, red in merge). Lower panel: transfected cells were incubated before fixation with MitoTracker red (left, red in merge). Notice the nuclear envelope labelling by NAT8L (arrows) and its reticular pattern in the cytoplasm with extensive co-localization with the ER marker (arrowheads), contrasting with full segregation from Golgi and mitochondrial labelling. Scale bars, 5 µm.

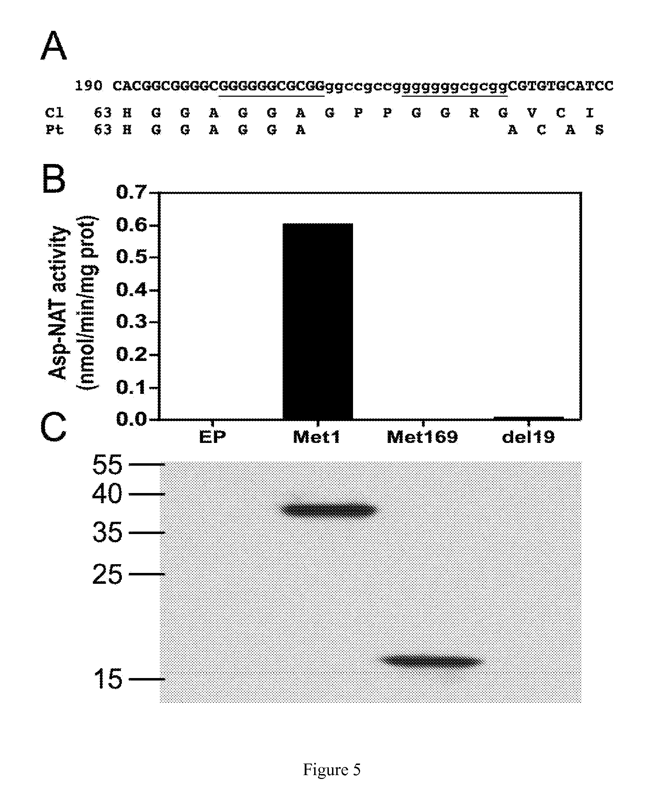

**Figure 5****. Mutation found in the patient with hypoacetylaspartia and its effect on the activity and expression of NAT8L**
   (*A*) Sequences of nucleotides 190-240 of the NAT8L open reading frame and deduced protein sequences in controls and in the patient. Nucleotide 1 corresponds to the adenine of the initiator ATG codon. The 19 nucleotides deleted in the patient are shown in small characters and the 11 bp repeats between which this deletion occurred is underlined. (*B* and *C*) HEK293T cells were transfected with an empty plasmid (EP) or a plasmid driving the expression of human wild-type NAT8L (Met1), the form with the 19-bp deletion (del19), and a shorter form corresponding to amino acids 169-302 (Met169). All proteins contained a C-terminal polyHis tag. (*B*) Asp-NAT activity in cell extracts; (*C*) Western blots with an anti-polyHis tag antibody.

### EXAMPLES

### Transfections of HEK 293T cells and western blotting

HEK 293T cells were cultured and transfected essentially as described by Rzem *et al.* (33) using the jetPEI procedure. After 48 h at 37°C, the cells were washed once with 5 ml of cold phosphate-buffered saline (PBS). They were scraped in 0.8 ml of 20 mM Hepes (pH 7.1) containing 5 µg/ml leupeptin and antipain, frozen in liquid nitrogen, thawed and lysed by vortexing. The extracts were incubated with 100 U/ml DNase I (from Sigma-Aldrich; prepared in 5 mM MgSO₄) for 1 hour on ice before use. Protein concentration was determined with the Bradford assay (34), using gamma-globulin as a standard. Western blots were performed as described (33) with a monoclonal pentaHis antibody (Qiagen) diluted 1:2000 in PBS containing 1 % bovine serum albumin. After washing, membranes were probed for 1 hour at room temperature with peroxidase-conjugated anti-mouse antibody. Detection was performed using the ECL Western blotting analysis system from GE Healthcare according to the manufacturer's protocol.

### N-acetylaspartate measurements by GC/MS and assay of aspartate N-acetyltransferase

Quantification of NAA in cell media was performed by isotope dilution gas chromatography-mass spectrometry as described by Kelley and Stamas (24) with *N*-[d₃]-acetylaspartate from VU Medical Center, Metabolic Laboratory (Dr H.J. ten Brink, Amsterdam, The Netherlands).

Asp-NAT was assayed radiochemically in a mixture (200 µl final volume) comprising 10 mM potassium phosphate, 20 mM Hepes, pH 7.1, 1 mM MgCl₂, 50 µM L-aspartate, 10⁵ cpm [U-¹⁴C]L-aspartate (GE Healthcare), 200 µM acetyl-CoA (Sigma-Aldrich), and 10 µl of a cell extract containing ≈ 5-10 mg protein/ml. After 20-40 min at 30°C, the reaction was arrested by a 5-min incubation at 80°C and 1 ml of 5 mM Hepes, pH 7.1 was added. The sample was centrifuged and the supernatant was applied on a 1-ml Dowex AG1X8 column (Cl⁻ form, 100-200 mesh, from Acros Organics) prepared in a Pasteur capillary pipette. The latter was washed successively with 2 ml 5 mM Hepes, pH 7.1, 5 ml 150 mM NaCl in the same buffer to elute unreacted aspartate, and 5 ml 300 mM NaCl to elute NAA. Radioactivity was counted in the presence of Ultima Gold^{™} (Perkin Elmer) in a liquid scintillation counter. The kinetic studies shown in Figure 3 were performed using washed pellets, obtained by centrifuging extracts for 20 min at 16 000 x g, resuspending them in the initial volume of extraction buffer, recentrifugation and resuspension in the original volume of extraction buffer.

### N-acetylaspartate measurements by GC/MS

Samples (0.2 ml) were treated with hydroxylamine hydrochloride in the presence of *N-*[d₃]-acetylaspartate as internal standard, and extracted with ethyl acetate followed by diethyl ether. After evaporation of the combined extracts under a stream of nitrogen, the residue was resuspended in acetonitrile, and derivatization was achieved with N,O-bis(trimethylsilyl)trifluoroacetamide/trimethylchlorosilane (BSTFA/TMCS). Analysis was performed by gas-chromatography (Hewlett-Packard 6890 series GC system equipped with a 30 m x 0.25 mm fused silica capillary column CP-SIL 8CB, Varian) with helium as carrier gas, coupled with a Hewlett-Packard 5973 Mass selective detector under electron-impact fragmentation. Mass spectrometric data were collected in the selected ion mode at m/z = 274 for native NAA, and m/z = 277 for synthetic isotopic NAA. Calibration curves were constructed by mixing 4.5 µg of the isotopic NAA with standard solution containing 0 - 6 µg of NAA, followed by drying under nitrogen and direct derivatization with the silylating agent.

### Mutation search

Genomic DNA analysis of the child with hypoacetylaspartia (16) was performed with informed consent of the legal parents. The 3 exons of the NAT8L gene were PCR-amplified (see Table 1), purified and sequenced directly on both strands. For the screening of 118 healthy controls, the region surrounding the deletion was amplified from genomic DNA with a specific couple of primers (Table 1). PCR products were directly loaded on a 2 % agarose gel containing 0.5 µg/ml ethidium bromide to distinguish wild-type (223 bp) and mutant (204 bp) alleles.

### Immunofluorescence in transfected CHO cells and infected primary neurons

CHO cells were propagated in DMEM/F-12 supplemented with 10 % fetal bovine serum (FBS) and antibiotics (100 U/ml penicillin and 100 µg/ml streptomycin). Cells were seeded at 50,000 cells/cm² in 24-well plates on glass coverslips coated with 25 µg/µl fibronectin (in PBS) and grown for one day (≈ 80 % confluency). Transfection with the NAT8L-pEF6*Myc*HisA plasmid or the PDIb-HA construct (27) was carried out at 0.5 µg total DNA and 1 µl lipofectamine/cm² at 37°C for 5 h followed by reincubation overnight in DMEM/F-12/10% FBS without antibiotics. After 24 h, cells were processed for immunofluorescence as described by Mettlen *et al.* (35).

For immunofluorescence experiments, neurons were seeded at 10⁵ cells/cm² on glass coverslips, fixed with paraformaldehyde 4% (v/v) for 15 min at room temperature and permeabilized thereafter with 1% Triton X100 (v/v) in PBS during 15 min. Non-specific immunostaining was prevented by 1 h incubation in a PBS solution containing 3% non-fat dry milk at room temperature.

For primary antibodies, we used mouse IgG₂ₐ anti-Myc-Tag (9B11) monoclonal antibody (1/500; Cell Signalling), mouse IgG₁ anti-His monoclonal antibody (1/50; Qiagen), rabbit affinity-purified specific antibodies against NAT8L (1/100), rabbit anti-HA antibodies (1/300), rat monoclonal antibody recognizing KDEL-bearing proteins (1/300; Abcam), either alone or combined with mouse IgG_{2b} monoclonal antibody to PDI (1/50; Abcam), rabbit MAP-2 primary antibody (B9; serum used at 1/1000 dilution) and Alexa-labelled secondary antibodies were from Molecular Probes/Invitrogen (each used at 5 µg/ml). For live mitochondria labelling, cells were incubated with MitoTracker-red (CMXRos; Molecular Probes; 250 nM in DMEM/F-12) at 37°C for 30 min followed by chase in MitoTracker-free medium for 30 min, before fixation-permeabilization. All preparations were mounted in Mowiol in the dark overnight and examined with a LSM 510 META confocal microscope (Zeiss, Jena, Germany) using a Plan-Apochromat 63X/1.4 oil DIC objective.

### Preparation of expression vectors

Human and mouse total brain cDNA were synthesized with random primers and 1.5-2.5 µg total RNA with the M-MuLV reverse transcriptase from Fermentas (St Leon-Rot, Germany) according to the manufacturer's instructions. FirstChoice^{®} human brain total RNA was from Applied Biosystems and mouse total brain RNA was prepared as described in (36).

The open reading frames of mouse *NAT14* (UCSC refseq gene NM_201355) and mouse *NAT8L* (UCSC refseq gene NM_001001985) were PCR-amplified using 2.5 U of PWO polymerase (Roche Applied Science) in the presence of 1.5 M betaine (Sigma-Aldrich) with mouse total brain cDNA as a template. For the non-tagged constructions, a 5'-primer (for primer sequences, see Table 1) containing the putative ATG preceded by a perfect Kozak sequence and a *Kpn*I site, and a 3'-primer containing the putative stop codon flanked by a *Xba*I site were used. For the C-tagged constructs, the 5'-primers were the same, while the putative stop codon in the 3'-primers was mutated and flanked by a *Xba*I site. For the N-tagged constructs, the 5'-primers were devoid of Kozak sequence and the 3'-primers were the same as for the wild-type amplifications. The PCR amplifications started with an initial denaturation step at 96°C for 3 min, followed by 35 cycles for 30 sec at 96°C, 1 min at 60°C and 1 min at 72°C, and ended with a final extension step at 72°C for 5 min. All PCRs reported in this paper were performed with this 5-step program except for site-directed mutagenesis (see below). PCR products of the expected size were obtained, subcloned into pBlueScript plasmid and checked by sequencing. The *Kpn*I/*Xba*I digested products were prepared from *E*. *coli* XL1blue strain (C-tagged constructs) or from *E. coli* JM110 strain (wild-type and N-tagged constructs) and inserted in pCMV5, pEF6MycHisA and pEF6HisB expression vectors for the wild-type, C-tagged and N-tagged constructs, respectively. For human NAT8L, amplifications were performed with 2.5 U of Pfu polymerase (Fermentas) in the presence of 1 M betaine, with 2 µl of human total brain cDNA as a template. The three types of construct were prepared as for the mouse sequences and checked by sequencing.

### Site-directed mutagenesis

Fifty ng of the pBlueScript vector containing the human NAT8L sequence designed to be expressed with a polyHis tag at its C-terminus were used as a template, in a 50 µl-PCR reaction containing 25 pmol of each primer (Table 1), 0.2 mM dNTP, 1 M betaine, and 2.5 U Pfu polymerase in 1 X Pfu buffer. All these primers were phosphorylated at their 5' end. This mixture was subjected to thermal cycling at 96°C for 30 sec for denaturation, followed by 30 sec at 55°C for annealing, and 5 min at 72°C for extension. A total of 20 cycles was used and the procedure was ended by 7 min at 72°C. For both mutants, 3 identical reactions were done and pooled at the purification step in 35 µl of 10 mM TrisHCl, pH 8.5. The purified PCR product (30 µl) was incubated overnight at 16°C with 5 U of T4 DNA ligase (Fermentas) in the presence of 5 % PEG 4000 in 50 µl of 1 X ligase buffer. The ligation product was then digested for 2 hours at 37°C with 20 U of *Dpn*I in order to eliminate the wild-type template. Fifteen µl of this mixture were used to transform 100 µl of *E*. *coli* XL1blue bacteria. The complete NAT8L coding region of all plasmids was sequenced to verify the introduced deletion and the absence of random mutations. The *Kpn*I/*Xba*I fragment was then subcloned in pEF6MycHisA vector and both mutants were expressed as polyHis/myc-tagged fusion proteins.

### Mutation search

The 3 exons of the NAT8L gene were PCR-amplified using 0.5 µM of forward and reverse primers (see Table 1) with 75 ng of genomic DNA as a template. These reactions (50 µl) were performed using Biotools DNA polymerase (Kordia Life Sciences, Leiden, the Netherlands) in its provided buffer, 0.2 mM dNTP, and 1 M betaine. PCR products were purified and sequenced directly on both strands by use of a CEQ2000 sequencer (Beckman) or by the sequencing service of Macrogen (Seoul, South Korea). For the screening of the 118 healthy controls, the region surrounding the deletion was amplified from genomic DNA with a specific couple of primers (see Table 1). PCR conditions were the same except that a concentration of 1.5 M betaine was used.

### Neuronal cultures and infection

Primary cultures of cortical neurons were prepared from 17-18 days old Wistar rat embryos as described previously (37). Brains were dissected, transferred to HBSS buffer medium (1 mM sodium pyruvate, 10 mM Hepes without Ca²⁺ and Mg²⁺), dissociated in the same medium containing 1.26 mM Ca²⁺ and Mg²⁺ (0.50 mM MgCl₂ and 0.41 mM MgSO₄) and centrifuged for 10 min at 200 g (room temperature). Cells were plated in culture dishes pre-treated with 10 µg/ml poly-L-lysine in PBS and cultured for 7 days in NEUROBASAL medium supplemented with 2% (v/v) B-27 medium and 0.5 mM L-glutamine prior to infection with recombinant lentivirus. The NAT8L-pEF6*Myc*HisA plasmid was utilized for construction of recombinant lentivirus, as previously described (38). After 7 days of culture, neurons were incubated in the presence of the lentivirus for 3 days before analysis.

### Specific antibodies against NAT8L

Antibodies were raised against two peptides of mouse NAT8L (C)NTAFRGLRQHPRTQLL (P44) and (C)MSVDSRFRGKGIAKALG (P45). These peptides were conjugated to keyhole limpet hemocyanin and injected together to rabbits 4 times at 2 weeks intervals. Serum was taken from the rabbits one month after the final injection of these peptides. IgG were isolated from the sera by affinity chromatography on protein-G (HITRAP protein G HP, GE Healthcare). IgG were bound to the column in PBS buffer and eluted with 50 mM glycine-HCl, pH 2.7, containing 150 mM NaCl. Collected fractions were immediately neutralized with 1 M Tris-HCl, pH 8. Antibodies specific to the P44 and P45 NAT8L peptides were then purified by affinity chromatography on columns coupled with the P44 or P45 peptide. Peptides were coupled through their amino-terminal cysteine to iodoacetamide immobilized on a crosslinked agarose support (Sulfolink, Thermo scientific PIERCE). IgG fractions were loaded on the peptide-coupled column in PBS. Antibodies bound to the column were eluted with 50 mM glycine-HCl, pH 2.7, containing 150 mM NaCl, and immediately neutralized with 1 M Tris-HCl, pH 8.

**Table 1**

| **Preparation of mouse NAT14 and NAT8L expression vectors** | | |
|---|---|---|
| couple 1 (non- tagged NAT 14) | SEQ ID NO : 15 | F :GGGTACCCCCGCCGCCACCATGGCCCCTAACCACCTG |
| | SEQ ID NO : 16 | R :TTCTAGATCACAGGTCTTTGCTGAATTCC |
| couple 2 (C-tagged NAT 14) | SEQ ID NO : 17 | F :GGGTACCCCCGCCGCCACCATGGCCCCTAACCACCTG |
| | SEQ ID NO : 18 | R :TTCTAGACAGGTCTTTGCTGAATTCCCTA |
| couple 3 (N-tagged NAT 14) | SEQ ID NO : 19 | F :GGGTACCCATGGCCCCTAACCACCTG |
| | SEQ ID NO : 20 | R :TTCTAGATCACAGGTCTTTGCTGAATTCC |
| couple 4 (non-tagged NAT8L) | SEQ ID NO : 21 | F :GGGTACCCCCGCCGCCACCATGCATTGTGGGCCTCCCG AC |
| | SEQ ID NO : 22 | R :GTCTAGATCACTCCTCGCGCAGCTGCAGG |
| couple 5 (C-tagged NAT8L) | SEQ ID NO : 23 | F :GGGTACCCCCGCCGCCACCATGCATTGTGGGCCTCCCG AC |
| | SEQ ID NO : 24 | R :GTCTAGACTCCTCGCGCAGCTGCAGG |
| couple 6 (N-tagged NAT8L) | SEQ ID NO : 25 | F :GGGTACCCATGCATTGTGGGCCTCCCGAC |
| | SEQ ID NO : 26 | R :GTCTAGATCACTCCTCGCGCAGCTGCAGG |

| **Site-directed mutagenesis** | | |
|---|---|---|
| couple 7 (del. nucl. 212 to 230) | SEQ ID NO : 27 | F :CGTGTGCATCCGCGAGTTCC |
| | SEQ ID NO : 28 | R :CCGCGCCCCCCGCCCCGCCG |
| couple 8 (del. nucl. 1 to 504) | SEQ ID NO : 29 | F :ATGGCGGACATCGAGCAGTACTA |
| | SEQ ID NO : 30 | R :GGTGGCGGCGGGGGTACCCA |

| **Mutation search** | | |
|---|---|---|
| couple 9 (exon 1) | SEQ ID NO : 7 | F :TTGCCCTGGGCCCGGCCGT |
| | SEQ ID NO : 13 | R :AGGCCGTGTTAGGGATGCGCTC |
| couple 10 (exon 1) | SEQ ID NO : 14 | F :TTGCCCTGGGCCCGGCCGT |
| | SEQ ID NO : 8 | R :CACGCCCATAGCCAGCCCTGC |
| couple 11 (exon 2) | SEQ ID NO : 9 | F :GTCCCTAGCGGGCTTCGCC |
| | SEQ ID NO : 10 | R :TGCCCTGAGGTCGCTGGAGTG |
| couple 12 (exon 3) | SEQ ID NO : 11 | F :TGTCTCACCCCAGAGGTGGAG |
| | SEQ ID NO : 12 | CCAATTGAGCGTCTGAAAGCAGG |
| couple 13 (controls screening) | SEQ ID NO : 31 | F :CAAGAAGGACGCGCTGCTCG |
| | SEQ ID NO : 32 | R :CATGATGCCGTCGTAGAAGATG |

| **Amplification of the human cDNA** | | |
|---|---|---|
| couple 14 | SEQ ID NO : 33 | F :CTGAGTTCACGGGACTGAAATC |
| | SEQ ID NO : 34 | R :CACCTTGCGGCTGTAGTAGTAG |
| couple 15 | SEQ ID NO : 35 | F :CTGAGTTCACGGGACTGAAATC |
| | SEQ ID NO : 36 | R :CCAGCGACTCGTAGAGCTTG |
| couple 16 | SEQ ID NO : 37 | F :GAGTTCATGCAGCGCGCCCT |
| | SEQ ID NO : 38 | R :CACCTTGCGGCTGTAGTAGTAG |
| couple 17 | SEQ ID NO : 39 | F :GAGTTCATGCAGCGCGCCCT |
| | SEQ ID NO : 40 | R :CCAGCGACTCGTAGAGCTTG |
| couple 18 | SEQ ID NO : 41 | F :GACATGGTCTGCGAGACGAAG |
| | SEQ ID NO : 42 | R :CACCTTGCGGCTGTAGTAGTAG |
| couple 19 | SEQ ID NO : 43 | F :GACATGGTCTGCGAGACGAAG |
| | SEQ ID NO : 44 | R :CCAGCGACTCGTAGAGCTTG |

| **Quantitative real time PCR** | | |
|---|---|---|
| couple 20 | SEQ ID NO : 45 | F :CCGCATCTTCTACGACGG |
| | SEQ ID NO : 46 | R :CGCACACTCCAGGTAGGC |
| couple 21 | SEQ ID NO : 47 | F :TACTACTACAGCCGCAAGG |
| | SEQ ID NO : 48 | R :GCGAGTCCACGGACATGC |

### Results

### Database searches

The characteristics of Asp-NAT are probably shared by only a limited number of mammalian gene products. Indeed, (1) this enzyme most likely belongs to a family of N-acetyltransferases; (2) it is mainly, if not exclusively, expressed in brain; (3) it is membrane-bound; (4) it should have close homologues only in species that synthesize NAA. We decided therefore to search mammalian genomes for genes encoding proteins with these characteristics.

A search in the human and mouse genomes with N-acetyltransferase (NAT) as a query yielded about 30 genes encoding N-acetyltransferases. These comprised a few well-identified sequences (e.g. N-acetyltransferases acting on arylamines, glucosamine-6-phosphate, glutamate, and spermine/spermidine) and a majority of putative N-acetyltransferases (i.e. hypothetical proteins showing sequence similarity to well characterized N-acetyltransferases) with undefined specificity. mRNA expression data of the Symatlas database (18) indicated that two of them were mainly present in brain: NAT14 and NAT8L. NAT14, also called Klp1 (K562 cell-derived leucine-zipper-like protein 1) is thought to be a transcription factor regulating the expression of coproporphyrinogen oxidase by binding to a promoter regulatory element (19). NAT8L, also designated Shati, is overexpressed in brain after administration of metamphetamine to mice (20). Both proteins showed at least one putative transmembrane domain. Close homologues (> 50 % sequence identity) of NAT8L and NAT14 were only found in vertebrates. Taken together, these findings indicated that both NAT8L and NAT14 were good candidates for Asp-NAT.

### Gene structure, tissue distribution, and protein sequence analysis of NAT8L

NAT8L is encoded by 3-exon genes on mouse chromosome 5 and human chromosome 4 (band 4p16.3). Previous PCR data obtained with mouse NAT8L (Shati) indicated that the mRNA encoding this protein is expressed not only in brain, but also in liver and kidneys (20). We therefore performed quantitative PCR amplification (as described in 21) on mouse tissue cDNA with couples of primers situated in exons 1 and 2, or in exons 2 and 3. Our findings indicated that NAT8L was amplified from brain cDNA and to a lesser extent from thymus or spleen cDNA. The abundance of the mRNA was calculated to be about ten-fold lower in these tissues than in brain. No specific amplification was observed with liver, kidney, lung, skeletal muscle, testis, and heart cDNA (results not shown).

The NAT8L cDNA was also amplified from human brain cDNA using primers situated in exons 1 and 3. Sequencing of the amplified products confirmed the deduced protein sequence registered under NCBI accession number EAW82541. No amplification could be observed with primers corresponding to a cDNA sequence encoding a protein (Swissprot Q8N9F0) that differs from human and mouse NAT8L in its first ≈ 90 residues. This cDNA contains in fact an exon from the upstream human gene (LOC401115 in human) fused to exons 2 and 3 of the NAT8L gene.

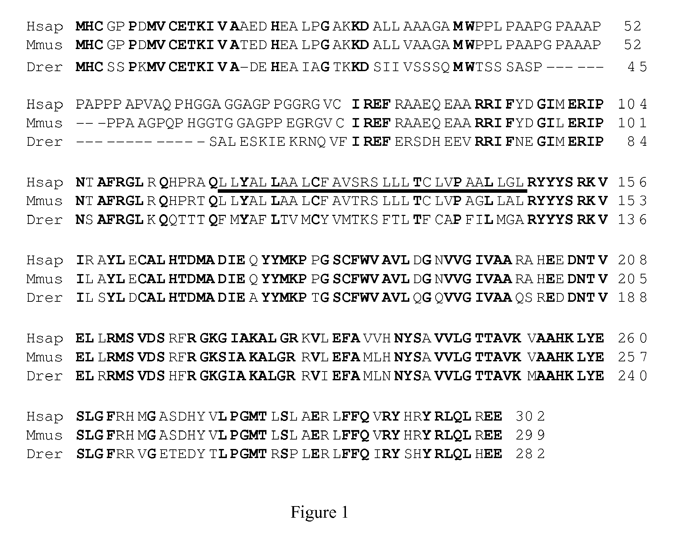

The NAT8L sequences of various species are aligned in Figure 1. As previously noted (20), the C-terminal region is homologous to N-acetyltransferases. NAT8Ls also comprise a conserved N-terminal sequence of 15 residues, a proline- and alanine-rich region of variable length (residues 40-73 in the human sequence), and a highly hydrophobic region of about 30 amino acids that just precedes the N-acetyltransferase domain.

Targeting prediction programmes [TargetP (22); PSort2 (23)] did not suggest that NAT8L had a mitochondrial propeptide or a signal peptide. It should be noted that exon 1 of human, mouse and rat NAT8L genes contains an in-frame stop codon that precedes the initiator ATG codon. This indicates that the protein sequence truly starts with the methionine shown as Met1 in the alignment. NAT14 is not described in detail as we show in the next section that it does not correspond to Asp-NAT.

### Identification of aspartate N-acetyltransferase as NAT8L

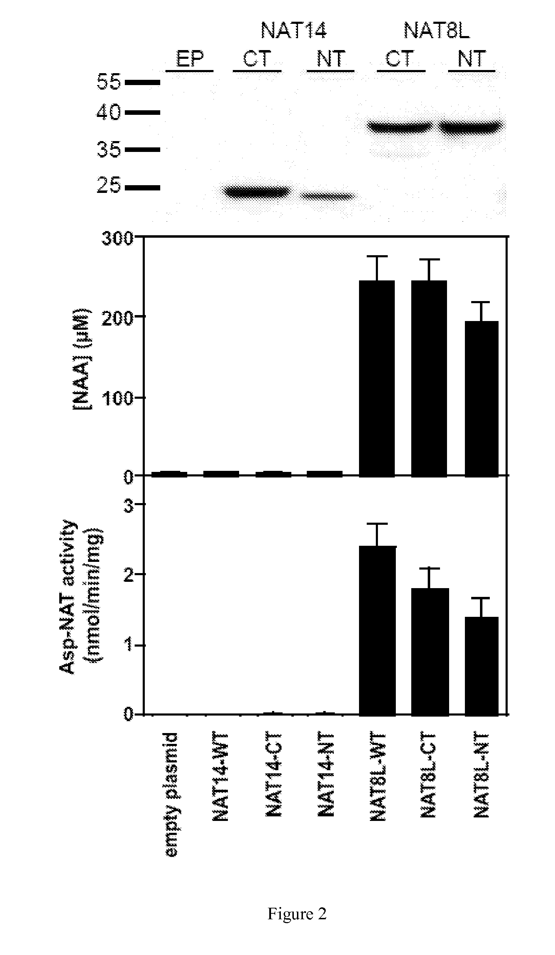

Both mouse NAT8L and NAT14 were overexpressed in HEK293T cells, as native proteins or as fusion proteins with a polyHis tag at the C- or the N-terminus. GC-MS analysis (24) of media collected after 48 hours indicated the presence of a product co-eluting with authentic NAA in medium from cells transfected with all NAT8L constructs, whereas this compound was below the detection limit (5 µM) in control cells (data not shown), or those transfected with NAT14 constructs. The fragmentation pattern of this product was identical with that of authentic NAA, with major fragments of m/z ratios 73, 147, 184, 245, 274 and 376 (24). Quantification of NAA by isotope dilution with the trideuterated compound (Figure 2 B) indicated that the concentration of NAA in the medium amounted to 200-250 µM in cells transfected with the different NAT8L constructs, corresponding to an amount of ≈ 300 nmol/mg cell protein. Analysis of cell extracts indicated that this compound was also present intracellularly at concentrations of about 60 nmol/mg protein (results not shown).

Asp-NAT activity was assayed in cell extracts as the acetyl-CoA-dependent conversion of [¹⁴C]L-aspartate to a more acidic product (Figure 2 *C*). In full agreement with the results mentioned above, this activity was observed in extracts of cells transfected with the three types of NAT8L constructs, but not in other extracts. Slightly lower activities were observed with the N-tagged and the C-tagged forms as compared to untagged NAT8L (59 % and 75 %, respectively). Western blot analysis with an anti-polyHis tag antibody (Figure 3 A) revealed that all His-tagged versions of NAT8L and NAT14 were expressed. This indicated that the absence of Asp-NAT activity in cells transfected with NAT14 was not due to lack of expression of this protein.

### Kinetic properties of NAT8L

The Asp-NAT activity was further characterized in extracts of cells expressing wild-type mouse NAT8L. The enzymatic activity was almost completely (> 90%) recovered in the pellet after a 20-min centrifugation of frozen-thawed extracts at 16.000 x g (not shown), indicating that it is membrane-bound. The kinetic studies were therefore performed on washed pellets, to eliminate soluble, potentially interfering enzymes. Detergents showed a biphasic effect on the activity (Figure 3A), increasing it at low concentration, presumably because they allowed access of the catalytic site to substrate, and being inhibitory at higher concentrations. Double-reciprocal plots of the saturation curve for aspartate was linear both in the absence and in the presence of 0.5 mM CHAPS, which increased Vₘₐₓ by ≈ 30 % without affecting the K_{M} (≈ 90 µM) for aspartate. With acetyl-CoA as varying substrate, the double-reciprocal plots were non-linear in the absence of detergent, consistent with part of the enzyme being poorly accessible to acetyl-CoA, but well in the presence of CHAPS (Figure 3 B). The K_{M} values amounted to ≈ 9 and 13 µM in the presence of 0.5 and 1 mM CHAPS, respectively.

[¹⁴C]Glutamate was also used by the enzyme, but with an about 50-fold lower affinity (K_{M} ≈ 5 mM) and a similar Vₘₐₓ compared to aspartate (not shown), in agreement with previous results (25, 26). Accordingly, the incorporation of [¹⁴C]-aspartate into NAA was inhibited by glutamate competitively with respect to aspartate with a Kᵢ of ≈ 6 mM (Figure 3 *C*). No inhibition was observed with other amino acids, indicating that the enzyme is specific. The enzyme was also inhibited by its reaction product NAA. This inhibition was essentially competitive with respect to aspartate (Kᵢ = 0.56 mM; Figure 3 D) and uncompetitive versus acetyl-CoA.

### Subcellular localization of NAT8L

Because of the controversy on the subcellular localization of Asp-NAT, it was of interest to check the localization of NAT8L in transfected cells. This was done by confocal microscopy analysis of CHO cells transfected with *myc*-tagged (at the C-terminus), his-tagged (at the N-terminus) or untagged NAT8L, and of neurons in primary culture infected with a lentiviral vector allowing the expression of myc-tagged NAT8L. Immunolabelling of *myc*-tagged NAT8L expressed in CHO cells or in primary neurons delineated the nuclear envelope (arrows in Figure 4) and produced a reticular pattern, strongly suggestive of the endoplasmic reticulum. This was confirmed by extensive co-localization of *myc-*tagged NAT8L with KDEL-bearing endoplasmic reticulum proteins (arrowheads in Figure 4). In contrast, the *myc*-tagged NAT8L signal was fully resolved from the Golgi complex, recognized by co-transfection with the PDIb-HA construct (27), and from mitochondria labelled *in vivo* by MitoTracker red (Figure 4). Targeting to the nuclear envelope and the endoplasmic reticulum was independent of epitope tagging, since NAT8L fused to a *myc-*tag at its C-terminus or to a polyHis-tag at its N-terminus showed a similar localization pattern to native NAT8L recognized with affinity-purified antibodies.

### Mutation of NAT8L in a patient with hypoacetylaspartia

To check whether NAT8L was mutated in the patient deficient in NAA, we amplified the three exons of the NAT8L gene and screened them for mutations. A 19-bp deletion in the homozygous form was observed in the first exon (Figure 5 A). This mutation leads to a change in the reading frame after amino acid 69 and it should therefore lead to no production of functional protein. To rule out the possibility that this mutation would still allow the production of an active protein by codon-slipping or by reinitiation at a later AUG codon, we prepared an expression vector for this mutated form (provided with a C-terminal his-tag) and transfected it in HEK293T cells. Extracts prepared from these cells did not contain any detectable Asp-NAT activity or material reacting with the anti-polyHis tag antibody, whereas extracts of cells transfected with the non-mutated human cDNA were enzymatically active and contained an immunoreactive band of the appropriate size (Figure 5 *B-C*).

Several human NAT8L protein entries (e.g. BAC04426.1) comprise only the last 134 AA of NAT8L. The sequence of this (putative) shorter protein is unaffected by the mutation and it was therefore important to verify if it displayed Asp-NAT activity. Transfection of a vector allowing the expression of this shorter form resulted in the expression of a polypeptide of the expected size (≈ 18 kDa) but to no detectable enzymatic activity (Figure *5 B-C*). Taken together, these findings indicated that the mutation found in the patient corresponds indeed to a null mutation.

The patient is an adopted child and no material was therefore available to check the presence of the mutation in the biological parents. The mutation found in the patient could be detected by PCR amplification of genomic DNA and gel electrophoresis. This assay allowed also the detection of the mutation in the heterozygous state, as indicated by amplifications performed on samples containing equal amounts of DNA from the patient and a control. The 19-bp deletion was not found in 118 control DNA samples either in the homozygous or the heterozygous state (results not shown).

### REFERENCES

1. Moffett JR, Ross B, Arun P, Madhavarao CN, Namboodiri AM (2007) N-Acetylaspartate in the CNS: from neurodiagnostics to neurobiology. Prog Neurobiol 81: 89-131.
2. Goldstein FB (1959) Biosynthesis ofN-acetyl-L-aspartic acid. J Biol Chem 234: 2702-2706.
3. Knizley H Jr (1967) The enzymatic synthesis of N-acetyl-L-aspartic acid by a water-insoluble preparation of a cat brain acetone powder. J Biol Chem 242: 4619-4622.
4. Goldstein FB (1969) The enzymatic synthesis of N-acetyl-L-aspartic acid by subcellular preparations of rat brain. J Biol Chem 244: 4257-4260.
5. Madhavarao CN, Namboodiri AM (2006) NAA synthesis and functional roles. Adv Exp Med Biol 576: 49-66.
6. Patel TB, Clark JB (1979) Synthesis of N-acetyl-L-aspartate by rat brain mitochondria and its involvement in mitochondrial/cytosolic carbon transport. Biochem J 184: 539-546.
7. Ariyannur PS, Madhavarao CN, Namboodiri AM (2008) N-acetylaspartate synthesis in the brain: Mitochondria vs. microsomes. Brain Res 1227: 34-41.
8. Lu ZH, Chakraborty G, Ledeen RW, Yahya D, Wu G (2004). N-Acetylaspartate synthase is bimodally expressed in microsomes and mitochondria of brain. Mol Brain Res 122: 71-78.
9. Chakraborty G, Mekala P, Yahya D, Wu G, Ledeen RW (2001) Intraneuronal N-acetylaspartate supplies acetyl groups for myelin lipid synthesis: evidence for myelin-associated aspartoacylase. J Neurochem 78: 736-45.
10. Tyson RL, Sutherland GR (1998) Labeling of N-acetylaspartate and N-acetylaspartylglutamate in rat neocortex, hippocampus and cerebellum from [1-13C]glucose. Neurosci Lett 251: 181-184.
11. Arun P, Madhavarao CN, Moffett JR, Namboodiri MA (2006) Regulation of N-acetylaspartate and N-acetylaspartylglutamate biosynthesis by protein kinase activators. J Neurochem 98: 2034-42.
12. Wroblewska B, Santi MR, Neale JH (1998) N-acetylaspartylglutamate activates cyclic AMP-coupled metabotropic glutamate receptors in cerebellar astrocytes. Glia 24: 172--179.
13. Moffett JR and Namboodiri AM (2006) Preface: a brief review of N-acetylaspartate. Adv Exp Med Biol 576: vii-xiii.
14. Matalon R, et al. (1988) Aspartoacylase deficiency and N-acetylaspartic aciduria in patients with Canavan disease. Am J Med Genet 29: 463-471.
15. Martin E, Capone A, Schneider J, Hennig J, Thiel T (2001) Absence of N-acetylaspartate in the human brain: impact on neurospectroscopy? Ann Neurol 49: 518-21.
16. Boltshauser E, et al. (2004) Follow-up of a child with hypoacetylaspartia. Neuropediatrics 35: 255-258.
17. Burlina AP, et al. (2006) Hypoacetylaspartia: clinical and biochemical follow-up of a patient. Adv Exp Med Biol 576: 283-287.
18. Su AI, et al. (2004) A gene atlas of the mouse and human protein-encoding transcriptomes. Proc Natl Acad Sci USA 101: 6062-7.
19. Takahashi S, et al. (2000) Cloning of a coproporphyrinogen oxidase promoter regulatory element binding protein. Biochem Biophys Res Commun 273: 596-602.
20. Niwa M, et al. (2007) A novel molecule "shati" is involved in methamphetamine-induced hyperlocomotion, sensitization, and conditioned place preference. J Neurosci 27: 7604-7615.
21. Maliekal P, Sokolova T, Vertommen D, Veiga-da-Cunha M, Van Schaftingen E (2007) Molecular identification of mammalian phosphopentomutase and glucose-1,6-bisphosphate synthase, two members of the alpha-D-phosphohexomutase family. J Biol Chem 282: 31844-31851.
22. Emanuelsson O, Nielsen H, Brunak S, von Heijne G (2000) Predicting subcellular localization of proteins based on their N-terminal amino acid sequence. J Mol Biol 300: 1005-16.
23. Nakai K, Horton P (1999) PSORT: a program for detecting sorting signals in proteins and predicting their subcellular localization. Trends Biochem Sci 24: 34-6.
24. Kelley RI and Stamas JN (1992) Quantification of N-acetyl-L-aspartic acid in urine by isotope dilution gas chromatography-mass spectrometry. J Inherit Metab Dis 15: 97-104.
25. Truckenmiller ME, Namboodiri MA, Brownstein MJ, Neale JH (1985) N-Acetylation of L-aspartate in the nervous system: differential distribution of a specific enzyme. J Neurochem 45: 1658-1662.
26. Madhavarao CN, Chinopoulos C, Chandrasekaran K, Namboodiri MA (2003) Characterization of the N-acetylaspartate biosynthetic enzyme from rat brain. J Neurochem 86: 824-835.
27. Raykhel I, et al. (2007) A molecular specificity code for the three mammalian KDEL receptors. J Cell Biol. 179: 1193-1204.
28. Lieberman EM, Achreja M, Urazaev AK (2006) Synthesis of N-acetylaspartylglutamate (NAAG) and N-acetylaspartate (NAA) in axons and glia of the crayfish medial giant nerve fiber. Adv Exp Med Biol 576: 303-315.
29. Verbalis JG (2006) Control of brain volume during hypoosmolality and hyperosmolality. Adv Exp Med Biol 576: 113-29.
30. Schuff N, et al. (2006) N-acetylaspartate as a marker of neuronal injury in neurodegenerative disease. Adv Exp Med Biol 576: 241-62.
31. Arun P, Madhavarao CN, Moffett JR, Namboodiri AM (2008) Antipsychotic drugs increase N-acetylaspartate and N-acetylaspartylglutamate in SH-SY5Y human neuroblastoma cells. J Neurochem 106: 1669-80.
32. Niwa M, et al. (2008) A novel molecule "shati" increases dopamine uptake via the induction of tumor necrosis factor-alpha in pheochromocytoma-12 cells. J Neurochem 107: 1697-1708.
33. Rzem R, et al. (2004) A gene encoding a putative FAD-dependent L-2-hydroxyglutarate dehydrogenase is mutated in L-2-hydroxyglutaric aciduria. Proc Natl Acad Sci USA 101: 16849-54.
34. Bradford MM (1976) A rapid and sensitive method for the quantitation of microgram quantities of protein utilizing the principle of protein-dye binding. Anal Biochem 72: 248-254.
35. Mettlen M, et al. (2006) Src triggers circular ruffling and macropinocytosis at the apical surface of polarized MDCK cells. Traffic 7: 589-603.
36. Maliekal P, Sokolova T, Vertommen D, Veiga-da-Cunha M, Van Schaftingen E (2007) Molecular identification of mammalian phosphopentomutase and glucose-1,6-bisphosphate synthase, two members of the alpha-D-phosphohexomutase family. J Biol Chem 282: 31844-31851.
37. Macq AF, et al. (1998) The long-term adenoviral expression of the human amyloid precursor protein shows different secretase activities in rat cortical neurons and astrocytes. J Biol Chem 273: 28931-28936.
38. Salmon P, Trono D (2007) Production and titration of lentiviral vectors. Curr Protoc Hum Genet Chapter 12: Unit 12.10.

## Claims

1. An isolated human aspartate-N-acetyltransferase having for sequence SEQ ID NO: 1 or an amino acid sequence having at least 90% identity with SEQ ID NO: 1.

2. An isolated murine aspartate-N-acetyltransferase having for sequence SEQ ID NO: 2 or an amino acid sequence having at least 90% identity with SEQ ID NO: 2.

3. An isolated rat aspartate-N-acetyltransferase having for sequence SEQ ID NO: 3 or an amino acid sequence having at least 90% identity with SEQ ID NO: 3.

4. An isolated nucleic acid sequence encoding the aspartate-N-acetyltransferase according to claims **1, 2 or 3** and fragments thereof.

5. The nucleic acid sequence according to claim 4, wherein said nucleic acid sequence encodes the human aspartate-N-acetyltransferase of claim 1 and has for sequence SEQ ID NO: 4.

6. A vector comprising a nucleic acid sequence according to claim **4 or 5**.

7. A host cell comprising a nucleic acid sequence according to any one of claim **4 or 5** or a vector according to claim **6**.

8. A non-human transgenic animal for aspartate-N-acetyltransferase.

9. An antibody that specifically recognises aspartate-N-acetyltransferase according to any one of claims **1** to **3**.

10. A method for screening or identifying compounds capable of modulating the aspartate-N-acetyltransferase enzyme activity, comprising
- contacting a compound to be tested with a model system expressing the aspartate-N-acetyltransferase enzyme having for sequence SEQ ID NO: 1, SEQ ID NO: 2 or SEQ ID NO: 3 in conditions allowing the enzymatic reaction, and
- determining the aspartate-N-acetyltransferase enzyme activity.

11. A method for determining whether a subject has or is at risk of having an aspartate-N-acetyltransferase enzyme-linked disease in a subject in need thereof, comprising detecting in a sample obtained from said subject genetic lesions or mutations in a nucleic acid sequence according to claim **4** or **5**.

12. The method according to claim **11**, wherein said aspartate-N-acetyltransferase enzyme activity-linked disease is selected in the group consisting of hypoacetylaspartia (aspartate N-acetyltransferase deficiency), epilepsy, canavan disease, leukodystrophies, Alzheimer's disease, multiple sclerosis, amyotrophic lateral sclerosis, stroke, AIDS encephalopathy and herpes encephalitis.

13. A kit comprising at least three couples of primers that allow the amplification of each of the three exons of the aspartate-N-acetyltransferase.

14. The kit according to claim **13,** comprising the couples of primers:
- exon 1; 5' part: TTGCCCTGGGCCCGGCCGT (SEQ ID NO: 11) and AGGCCGTGTTAGGGATGCGCTC (SEQ ID NO : 12);
- exon 1 ; 3' part: TTGCCCTGGGCCCGGCCGT (SEQ ID NO : 13) and CACGCCCATAGCCAGCCCTGC (SEQ ID NO : 14);
- exon 2: GTCCCTAGCGGGCTTCGCC (SEQ ID NO: 7) and TGCCCTGAGGTCGCTGGAGTG (SEQ ID NO : 8);
- exon 3: TGTCTCACCCCAGAGGTGGAG (SEQ ID NO: 9) and CCAATTGAGCGTCTGAAAGCAGG (SEQ ID NO:10)

15. Aspartate for use in treating aspartate-N-acetyltransferase linked disease wherein the level ofNAA is decreased.

16. N-acetylaspartate (NAA) or precursor thereof for use in treating aspartate-N-acetyltransferase linked disease wherein no NAA is produced.


SEQUENCE LISTING

<110>  Université Catholique de Louvain
       VanSchaftingen, Emile

<120>  ASPARTATE-N-ACETYLTRANSFERASE ENZYME, DIAGNOSTIC METHOD AND
       THERAPEUTIC METHOD.

<130>  BEP090407

<160>  48

<170>  PatentIn version 3.3

<210>  1
<211>  302
<212>  PRT
<213>  homo sapiens

<400>  1

Met His Cys Gly Pro Pro Asp Met Val Cys Glu Thr Lys Ile Val Ala
1               5                   10                  15

Ala Glu Asp His Glu Ala Leu Pro Gly Ala Lys Lys Asp Ala Leu Leu
            20                  25                  30

Ala Ala Ala Gly Ala Met Trp Pro Pro Leu Pro Ala Ala Pro Gly Pro
        35                  40                  45

Ala Ala Ala Pro Pro Ala Pro Pro Pro Ala Pro Val Ala Gln Pro His
    50                  55                  60

Gly Gly Ala Gly Gly Ala Gly Pro Pro Gly Gly Arg Gly Val Cys Ile
65                  70                  75                  80

Arg Glu Phe Arg Ala Ala Glu Gln Glu Ala Ala Arg Arg Ile Phe Tyr
                85                  90                  95

Asp Gly Ile Met Glu Arg Ile Pro Asn Thr Ala Phe Arg Gly Leu Arg
            100                 105                 110

Gln His Pro Arg Ala Gln Leu Leu Tyr Ala Leu Leu Ala Ala Leu Cys
        115                 120                 125

Phe Ala Val Ser Arg Ser Leu Leu Leu Thr Cys Leu Val Pro Ala Ala
    130                 135                 140

Leu Leu Gly Leu Arg Tyr Tyr Tyr Ser Arg Lys Val Ile Arg Ala Tyr
145                 150                 155                 160

Leu Glu Cys Ala Leu His Thr Asp Met Ala Asp Ile Glu Gln Tyr Tyr
                165                 170                 175

Met Lys Pro Pro Gly Ser Cys Phe Trp Val Ala Val Leu Asp Gly Asn
            180                 185                 190

Val Val Gly Ile Val Ala Ala Arg Ala His Glu Glu Asp Asn Thr Val
        195                 200                 205

Glu Leu Leu Arg Met Ser Val Asp Ser Arg Phe Arg Gly Lys Gly Ile
    210                 215                 220

Ala Lys Ala Leu Gly Arg Lys Val Leu Glu Phe Ala Val Val His Asn
225                 230                 235                 240

Tyr Ser Ala Val Val Leu Gly Thr Thr Ala Val Lys Val Ala Ala His
                245                 250                 255

Lys Leu Tyr Glu Ser Leu Gly Phe Arg His Met Gly Ala Ser Asp His
            260                 265                 270

Tyr Val Leu Pro Gly Met Thr Leu Ser Leu Ala Glu Arg Leu Phe Phe
        275                 280                 285

Gln Val Arg Tyr His Arg Tyr Arg Leu Gln Leu Arg Glu Glu
    290                 295                 300

<210>  2
<211>  299
<212>  PRT
<213>  Mus musculus

<400>  2

Met His Cys Gly Pro Pro Asp Met Val Cys Glu Thr Lys Ile Val Ala
1               5                   10                  15

Thr Glu Asp His Glu Ala Leu Pro Gly Ala Lys Lys Asp Ala Leu Leu
            20                  25                  30

Val Ala Ala Gly Ala Met Trp Pro Pro Leu Pro Ala Ala Pro Gly Pro
        35                  40                  45

Ala Ala Ala Pro Pro Pro Ala Ala Gly Pro Gln Pro His Gly Gly Thr
    50                  55                  60

Gly Gly Ala Gly Pro Pro Glu Gly Arg Gly Val Cys Ile Arg Glu Phe
65                  70                  75                  80

Arg Ala Ala Glu Gln Glu Ala Ala Arg Arg Ile Phe Tyr Asp Gly Ile
                85                  90                  95

Leu Glu Arg Ile Pro Asn Thr Ala Phe Arg Gly Leu Arg Gln His Pro
            100                 105                 110

Arg Thr Gln Leu Leu Tyr Ala Leu Leu Ala Ala Leu Cys Phe Ala Val
        115                 120                 125

Thr Arg Ser Leu Leu Leu Thr Cys Leu Val Pro Ala Gly Leu Leu Ala
    130                 135                 140

Leu Arg Tyr Tyr Tyr Ser Arg Lys Val Ile Leu Ala Tyr Leu Glu Cys
145                 150                 155                 160

Ala Leu His Thr Asp Met Ala Asp Ile Glu Gln Tyr Tyr Met Lys Pro
                165                 170                 175

Pro Gly Ser Cys Phe Trp Val Ala Val Leu Asp Gly Asn Val Val Gly
            180                 185                 190

Ile Val Ala Ala Arg Ala His Glu Glu Asp Asn Thr Val Glu Leu Leu
        195                 200                 205

Arg Met Ser Val Asp Ser Arg Phe Arg Gly Lys Gly Ile Ala Lys Ala
    210                 215                 220

Leu Gly Arg Arg Val Leu Glu Phe Ala Met Leu His Asn Tyr Ser Ala
225                 230                 235                 240

Val Val Leu Gly Thr Thr Ala Val Lys Val Ala Ala His Lys Leu Tyr
                245                 250                 255

Glu Ser Leu Gly Phe Arg His Met Gly Ala Ser Asp His Tyr Val Leu
            260                 265                 270

Pro Gly Met Thr Leu Ser Leu Ala Glu Arg Leu Phe Phe Gln Val Arg
        275                 280                 285

Tyr His Arg Tyr Arg Leu Gln Leu Arg Glu Glu
    290                 295

<210>  3
<211>  299
<212>  PRT
<213>  Rattus norvegicus

<400>  3

Met His Cys Gly Pro Pro Asp Met Val Cys Glu Thr Lys Ile Val Ala
1               5                   10                  15

Thr Glu Asp His Glu Ala Leu Pro Gly Ala Lys Lys Asp Ala Leu Leu
            20                  25                  30

Ala Ala Ala Gly Ala Met Trp Pro Pro Leu Pro Ala Ala Pro Gly Pro
        35                  40                  45

Ala Ala Ala Pro Pro Pro Ala Ala Gly Pro Gln Pro His Gly Gly Thr
    50                  55                  60

Gly Gly Ala Gly Pro Pro Glu Gly Arg Gly Val Cys Ile Arg Glu Phe

65                  70                  75                  80

Arg Ala Ala Glu Gln Glu Ala Ala Arg Arg Ile Phe Tyr Asp Gly Ile
                85                  90                  95

Leu Glu Arg Ile Pro Asn Thr Ala Phe Arg Gly Leu Arg Gln His Pro
            100                 105                 110

Arg Thr Gln Leu Leu Tyr Ala Leu Leu Ala Ala Leu Cys Phe Ala Val
        115                 120                 125

Thr Arg Ser Leu Leu Leu Thr Cys Leu Val Pro Ala Gly Leu Leu Ala
    130                 135                 140

Leu Arg Tyr Tyr Tyr Ser Arg Lys Val Ile Leu Ala Tyr Leu Glu Cys
145                 150                 155                 160

Ala Leu His Thr Asp Met Ala Asp Ile Glu Gln Tyr Tyr Met Lys Pro
                165                 170                 175

Pro Gly Ser Cys Phe Trp Val Ala Val Leu Asp Gly Asn Val Val Gly
            180                 185                 190

Ile Val Ala Ala Arg Ala His Glu Glu Asp Asn Thr Val Glu Leu Leu
        195                 200                 205

Arg Met Ser Val Asp Ser Arg Phe Arg Gly Lys Gly Ile Ala Lys Ala
    210                 215                 220

Leu Gly Arg Arg Val Leu Glu Phe Ala Met Leu His Asn Tyr Ser Ala
225                 230                 235                 240

Val Val Leu Gly Thr Thr Ala Val Lys Val Ala Ala His Lys Leu Tyr
                245                 250                 255

Glu Ser Leu Gly Phe Arg His Met Gly Ala Ser Asp His Tyr Val Leu
            260                 265                 270

Pro Gly Met Thr Leu Ser Leu Ala Glu Arg Leu Phe Phe Gln Val Arg
        275                 280                 285

Tyr His Arg Tyr Arg Leu Gln Leu Arg Glu Glu
    290                 295

<210>  4
<211>  900
<212>  DNA
<213>  homo sapiens

<400>  4
atgcattgtg ggcctcccga catggtctgc gagacgaaga tcgtggctac ggaggaccat      60

gaggcactgc cgggggccaa gaaggacgcg ctgctcgtcg ccgccggagc catgtggccc     120

ccgctgcccg ccgcgcccgg gccggccgcc gcacccccac ccgcggcagg tccccagccc 180

cacggaggca ccgggggcgc ggggccgccg gaggggcgcg gcgtgtgcat ccgcgagttc 240

cgcgcagccg agcaggaggc ggcgcgccgc atcttctacg acggcatctt ggagcgcatc 300

cccaacacgg ctttccgcgg cctgcggcag cacccgcgca cccaactgct ctacgccctg 360

ctggccgccc tctgttttgc tgtgacccgc tcactgctgc tgacatgcct ggtgccggcc 420

gggctcctgg ccctgcgcta ctactacagc cgcaaggtga ttctggccta cctggagtgt 480

gcgctgcaca cggacatggc tgacattgag cagtactaca tgaagccacc tggttcctgt 540

ttctgggtgg ctgtgctgga cggcaacgtt gtgggcatcg tggccgcacg ggcccatgag 600

gaggacaaca cagtggagct gctccgcatg tccgtggact cgcgtttccg cggaaaaggc 660

atcgccaagg ctctgggcag gagggtgttg gagtttgcca tgctgcacaa ctactctgca 720

gtggtactgg gcaccacagc cgttaaggtg gccgcccaca agctctatga gtcactgggc 780

ttcagacaca tgggcgcgag tgatcactac gtgctgcctg gcatgaccct ctcgctggcc 840

gagcgcctct tcttccaggt ccgctaccac cgctaccgcc tgcagctgcg cgaggagtga 900

<210> 5
<211> 900
<212> DNA
<213> Mus musculus

<400> 5
atgcattgtg ggcctcccga catggtctgc gagacgaaga tcgtggctac ggaggaccat 60

gaggcactgc cgggggccaa gaaggacgcg ctgctcgtcg ccgccggagc catgtggccc 120

ccgctgcccg ccgcgcccgg gccggccgcc gcacccccac ccgcggcagg tccccagccc 180

cacggaggca ccgggggcgc ggggccgccg gaggggcgcg gcgtgtgcat ccgcgagttc 240

cgcgcagccg agcaggaggc ggcgcgccgc atcttctacg acggcatctt ggagcgcatc 300

cccaacacgg ctttccgcgg cctgcggcag cacccgcgca cccaactgct ctacgccctg 360

ctggccgccc tctgttttgc tgtgacccgc tcactgctgc tgacatgcct ggtgccggcc 420

gggctcctgg ccctgcgcta ctactacagc cgcaaggtga ttctggccta cctggagtgt 480

gcgctgcaca cggacatggc tgacattgag cagtactaca tgaagccacc tggttcctgt 540

ttctgggtgg ctgtgctgga cggcaacgtt gtgggcatcg tggccgcacg ggcccatgag 600

gaggacaaca cagtggagct gctccgcatg tccgtggact cgcgtttccg cggaaaaggc 660

atcgccaagg ctctgggcag gagggtgttg gagtttgcca tgctgcacaa ctactctgca 720

gtggtactgg gcaccacagc cgttaaggtg gccgcccaca agctctatga gtcactgggc 780

ttcagacaca tgggcgcgag tgatcactac gtgctgcctg gcatgaccct ctcgctggcc 840

gagcgcctct tcttccaggt ccgctaccac cgctaccgcc tgcagctgcg cgaggagtga 900

<210> 6
<211> 903
<212> DNA

<213>  Rattus norvegicus

<400>  6
atgcattgtg ggcctcccga catggtctgc gagacgaaga tcgtggctac ggaggaccat      60

gaggcactgc cgggggccaa gaaggacgcg ctgctcgccg ccgccggagc catgtggccc     120

ccgctgcccg ccgcgcccgg gccggctgcc gcacccccac ccgcggcagg tccccagccc     180

cacggaggca ccgggggcgc ggggccgccg gaggggcgcg gcgtgtgcat ccgcgagttc     240

cgcgcagccg agcaggaggc ggcgcgccgc atcttctacg acggcatctt ggagcgcatc     300

cccaacacgg ctttccgagg cctgcggcag cacccgcgca cccagctgct ctacgccctg     360

ctggcggccc tctgttttgc tgtgacccgc tcactgctgc tgacatgcct ggtgccggcc     420

gggctcctgg ccctgcgcta ctactacagt cgcaaggtga ttctggccta cctggagtgt     480

gcgctgcaca cggacatggc tgacattgaa cagtactaca tgaagccacc tggttcctgt     540

ttctgggtgg ctgtgctgga cggcaatgtt gtgggcatcg tggcggctcg ggcccatgag     600

gaagacaaca cagtggagct gctccgcatg tccgtggact cgcgcttccg cggcaaaggc     660

atcgccaagg ccctgggcag gagggtgttg gagtttgcca tgctgcacaa ctactctgca     720

gtggtactgg gcaccacagc cgttaaggtg gccgcccaca agctctatga gtcactgggc     780

ttcagacaca tgggcgcgag tgatcactac gtgctgcctg gcatgaccct ctcgctggcc     840

gagcgcctct tcttccaggt ccgctaccac cgctaccgcc tgcagctgcg cgaggagtga     900

cca                                                                   903


<210>  7
<211>  19
<212>  DNA
<213>  artificial

<220>
<223>  sense primer aspartate-N-acetyltransferase (exon 1)

<400>  7
ttgccctggg cccggccgt                                                   19


<210>  8
<211>  21
<212>  DNA
<213>  artificial

<220>
<223>  antisense primer aspartate-N-acetyltransferase (exon 1)

<400>  8
cacgcccata gccagccctg c                                                21


<210>  9
<211>  19
<212>  DNA
<213>  artificial

<220>
<223>  sense primer aspartate-N-acetyltransferase (exon 2)

<400>  9
gtccctagcg ggcttcgcc                                                    19


<210>  10
<211>  21
<212>  DNA
<213>  artificial

<220>
<223>  antisense primer aspartate-N-acetyltransferase (exon 2)

<400>  10
tgccctgagg tcgctggagt g                                                 21


<210>  11
<211>  21
<212>  DNA
<213>  artificial

<220>
<223>  sense primer aspartate-N-acetyltransferase (exon 3)

<400>  11
tgtctcaccc cagaggtgga g                                                 21


<210>  12
<211>  23
<212>  DNA
<213>  artificial

<220>
<223>  antisense primer aspartate-N-acetyltransferase (exon 3)

<400>  12
ccaattgagc gtctgaaagc agg                                               23


<210>  13
<211>  22
<212>  DNA
<213>  artificial

<220>
<223>  antisense primer aspartate-N-acetyltransferase (exon 1, 5' part)

<400>  13
aggccgtgtt agggatgcgc tc                                                22


<210>  14
<211>  19
<212>  DNA
<213>  artificial

<220>
<223>  sense primer aspartate-N-acetyltransferase (exon 1, 3' part)

<400>  14
ttgccctggg cccggccgt                                                    19


<210>  15
<211>  37
<212>  DNA
<213>  artificial

<220>
<223>  forward primer non-tagged NAT14

<400>  15
gggtaccccc gccgccacca tggcccctaa ccacctg                                37


<210>  16
<211>  29
<212>  DNA
<213>  artificial

<220>
<223>  reverse primer non-tagged NAT14

<400>  16
ttctagatca caggtctttg ctgaattcc                                         29


<210>  17
<211>  37
<212>  DNA
<213>  artificial

<220>
<223>  forward primer C-tagged NAT14

<400>  17
gggtaccccc gccgccacca tggcccctaa ccacctg                                37


<210>  18
<211>  29
<212>  DNA
<213>  artificial

<220>
<223>  reverse primer C-tagged NAT14

<400>  18
ttctagacag gtctttgctg aattcccta                                         29


<210>  19
<211>  26
<212>  DNA
<213>  artificial

<220>
<223>  forward primer N-tagged NAT14

<400>  19
gggtacccat ggcccctaac cacctg                                            26


<210>  20
<211>  29
<212>  DNA
<213>  artificial

<220>
<223>  reverse primer N-tagged NAT14

<400>  20
ttctagatca caggtctttg ctgaattcc                                         29

<210>  21
<211>  40
<212>  DNA
<213>  artificial

<220>
<223>  forward primer non-tagged NAT8L

<400>  21
gggtaccccc gccgccacca tgcattgtgg gcctcccgac                              40


<210>  22
<211>  29
<212>  DNA
<213>  artificial

<220>
<223>  reverse primer non-tagged NAT8L

<400>  22
gtctagatca ctcctcgcgc agctgcagg                                          29


<210>  23
<211>  40
<212>  DNA
<213>  artificial

<220>
<223>  forward primer C-tagged NAT8L

<400>  23
gggtaccccc gccgccacca tgcattgtgg gcctcccgac                              40


<210>  24
<211>  26
<212>  DNA
<213>  artificial

<220>
<223>  reverse primer C-tagged NAT8L

<400>  24
gtctagactc ctcgcgcagc tgcagg                                             26


<210>  25
<211>  29
<212>  DNA
<213>  artificial

<220>
<223>  forward primer N-tagged NAT8L

<400>  25
gggtacccat gcattgtggg cctcccgac                                          29


<210>  26
<211>  29
<212>  DNA
<213>  artificial

<220>
<223>  reverse primer N-tagged NAT8L

<400>  26
gtctagatca ctcctcgcgc agctgcagg                                          29


<210>  27
<211>  20
<212>  DNA
<213>  artificial

<220>
<223>  forward primer del. nucl. 212 to 230 NAT8L

<400>  27
cgtgtgcatc cgcgagttcc                                                    20


<210>  28
<211>  20
<212>  DNA
<213>  artificial

<220>
<223>  reverse primer del. nucl. 212 to 230 NAT8L

<400>  28
ccgcgccccc cgccccgccg                                                    20


<210>  29
<211>  23
<212>  DNA
<213>  artificial

<220>
<223>  forward primer del. nucl. 1 to 504

<400>  29
atggcggaca tcgagcagta cta                                                23


<210>  30
<211>  20
<212>  DNA
<213>  artificial

<220>
<223>  reverse primer del. nucl. 1 to 504

<400>  30
ggtggcggcg ggggtaccca                                                    20


<210>  31
<211>  20
<212>  DNA
<213>  artificial

<220>
<223>  forward primer controls screening

<400>  31
caagaaggac gcgctgctcg                                                    20


<210>  32
<211>  22
<212>  DNA
<213>  artificial

<220>
<223>  reverse primer controls screening

<400>  32
catgatgccg tcgtagaaga tg                                              22


<210>  33
<211>  22
<212>  DNA
<213>  artificial

<220>
<223>  forward primer for NAT8L cDNA amplification

<400>  33
ctgagttcac gggactgaaa tc                                              22


<210>  34
<211>  22
<212>  DNA
<213>  artificial

<220>
<223>  reverse primer for NAT8L cDNA amplification

<400>  34
caccttgcgg ctgtagtagt ag                                              22


<210>  35
<211>  22
<212>  DNA
<213>  artificial

<220>
<223>  forward primer for NAT8L cDNA amplification

<400>  35
ctgagttcac gggactgaaa tc                                              22


<210>  36
<211>  20
<212>  DNA
<213>  artificial

<220>
<223>  reverse primer for NAT8L cDNA amplification

<400>  36
ccagcgactc gtagagcttg                                                 20


<210>  37
<211>  20
<212>  DNA
<213>  artificial

<220>
<223>  forward primer for NAT8L cDNA amplification

<400>  37
gagttcatgc agcgcgccct                                                 20

<210>  38
<211>  22
<212>  DNA
<213>  artificial

<220>
<223>  reverse primer for NAT8L cDNA amplification

<400>  38
caccttgcgg ctgtagtagt ag                                                22


<210>  39
<211>  20
<212>  DNA
<213>  artificial

<220>
<223>  forward primer for NAT8L cDNA amplification

<400>  39
gagttcatgc agcgcgccct                                                   20


<210>  40
<211>  20
<212>  DNA
<213>  artificial

<220>
<223>  reverse primer for NAT8L cDNA amplification

<400>  40
ccagcgactc gtagagcttg                                                   20


<210>  41
<211>  21
<212>  DNA
<213>  artificial

<220>
<223>  forward primer for NAT8L cDNA amplification

<400>  41
gacatggtct gcgagacgaa g                                                 21


<210>  42
<211>  22
<212>  DNA
<213>  artificial

<220>
<223>  reverse primer for NAT8L cDNA amplification

<400>  42
caccttgcgg ctgtagtagt ag                                                22


<210>  43
<211>  21
<212>  DNA
<213>  artificial

<220>
<223>  forward primer for NAT8L cDNA amplification

<400>  43
gacatggtct gcgagacgaa g                                                21

<210>  44
<211>  20
<212>  DNA
<213>  artificial

<220>
<223>  reverse primer for NAT8L cDNA amplification

<400>  44
ccagcgactc gtagagcttg                                                  20

<210>  45
<211>  18
<212>  DNA
<213>  artificial

<220>
<223>  forward primer for NAT8L quantitative real time PCR

<400>  45
ccgcatcttc tacgacgg                                                    18

<210>  46
<211>  17
<212>  DNA
<213>  artificial

<220>
<223>  reverse primer for NAT8L quantitative real time PCR

<400>  46
cgcacactcc aggtagg                                                     17

<210>  47
<211>  19
<212>  DNA
<213>  artificial

<220>
<223>  forward primer for NAT8L quantitative real time PCR

<400>  47
tactactaca gccgcaagg                                                   19

<210>  48
<211>  18
<212>  DNA
<213>  artificial

<220>
<223>  reverse primer for NAT8L quantitative real time PCR

<400>  48
gcgagtccac ggacatgc                                                    18